(19) Europäisches Patentamt
European Patent Office
Office européen des brevets

(11) Numéro de publication: **0 254 627 B1**

(12) **FASCICULE DE BREVET EUROPEEN**

(45) Date de publication de fascicule du brevet: **17.06.92**

(51) Int. Cl.[5]: **C07D 295/12**, C07D 207/40, C07D 209/46, C07D 235/26, C07D 239/90, A61K 31/495

(21) Numéro de dépôt: **87401614.0**

(22) Date de dépôt: **09.07.87**

Le dossier contient des informations techniques présentées postérieurement au dépôt de la demande et ne figurant pas dans le présent fascicule.

(54) **Dérivés de la benzhydryloxyéthylpipérazine, procédés d'obtention et compositions pharmaceutiques les contenant.**

(30) Priorité: **10.07.86 FR 8610114**

(43) Date de publication de la demande:
**27.01.88 Bulletin 88/04**

(45) Mention de la délivrance du brevet:
**17.06.92 Bulletin 92/25**

(84) Etats contractants désignés:
**AT BE CH DE ES FR GB GR IT LI LU NL SE**

(56) Documents cités:
**EP-A- 0 099 148** **EP-A- 0 113 226**
**EP-A- 0 129 207** **EP-A- 0 157 420**
**FR-A- 2 235 691** **FR-A- 2 276 824**
**FR-A- 2 350 100** **FR-A- 2 374 318**

(73) Titulaire: **Société anonyme: LES LABORATOIRES MERAM**
**4 Bld Malesherbes**
**F-75008 Paris(FR)**

(72) Inventeur: **Buzas, André**
**25, Route de Versailles**
**F-91570 Bievres(FR)**
Inventeur: **Merour, Jean-Yves**
**216, Allée des Pervenches**
**F-45160 Olivet(FR)**
Inventeur: **Ollivier, Roland**
**94, Rue de Fauvettes**
**F-45160 Olivet(FR)**

(74) Mandataire: **Gillard, Marie-Louise et al**
**Cabinet Beau de Loménie 55, Rue d'Amsterdam**
**F-75008 Paris(FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouveaux dérivés de la benzhydryloxyéthyl-pipérazine. Elle concerne également les sels d'addition d'acides de tels dérivés. De plus, elle a aussi pour objet les procédés d'obtention de ces dérivés et les compositions pharmaceutiques les contenant.

On connaît déja de nombreux dérivés de benzhydrol présentant des activités thérapeutiques diverses. Toutefois, ces dérivés de formule générale I :

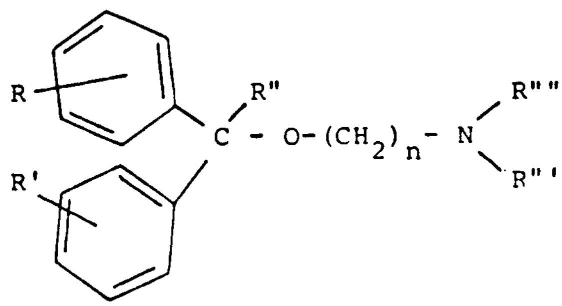

dans laquelle R , R′ représentent, indépendamment les uns des autres, un hydrogène, un alkyle, un alkoxy ou un halogène, R″ est soit un hydrogène, soit un alkyle,
le motif NR''''R''' représente un groupement dialkylamino, alkylamino, pyrrolidino, pipéridino, morpholino ou pipérazino substitué,
présentent une action sur le système nerveux central et en particulier une composante sédative. A cet effet, on peut se référer aux documents ci-après:

- BURGER'S MEDICINAL CHEMISTRY 4ème Edition, tome III, Manfred WOLFF, p.559-564 WILEY N.Y. Eds.
- J. M. MELON et A. BUZAS Fr 74.23.262 et Fr 76.I3.592
- GOOTJES J. et Coll. E.P. 0.099.I48.

Des dérivés de benzhydrylpipérazine de formule :

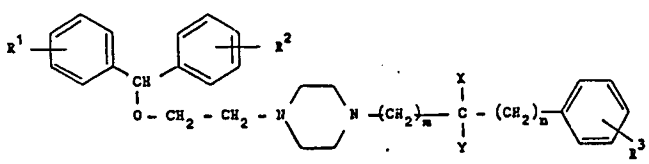

à activité dopaminergique sont décrits dans la demande EP O 099 148.

On connaît également des dérivés substitués de la pipérazine de formule :

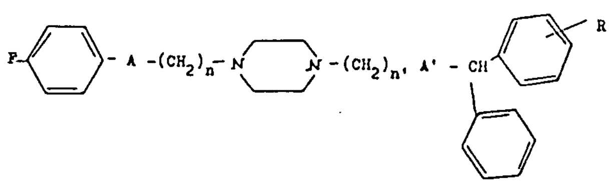

présentant des propriétés anti-émétiques et spasmolytiques. De tels dérivés sont décrits dans le brevet FR 76 13 592.

Des inhibiteurs de lipoxygénase présentant une activité anti- allergique de formule :

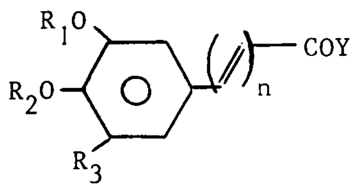

dans laquelle Y peut représenter un groupe

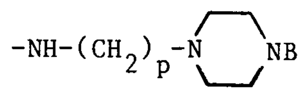

sont également décrits dans la demande EP O 157 420.

Par ailleurs, des dérivés phénylalkyl(pipérazinyl ou tromopipérazinyl)-propyl-(urées ou thiourées) substitués de formule :

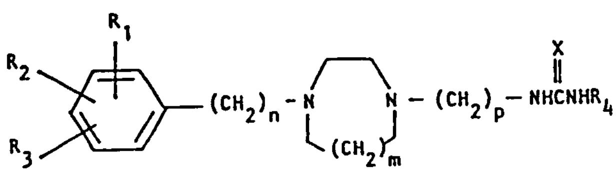

sont décrits dans la demande EP 0 129 207. Ces composés présentent des activités anti-histaminiques.
Des dérivés de la 1-benzydrylpipérazine à activité anti-allergique de formule :

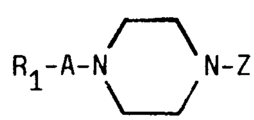

dans laquelle

- A est alkylène inférieur
- Z est benzhydryle éventuellement substitué par un halogène
- $R_1$ est amino, aryl, pyridyl, acyl ou acylamino, aryl et pyridyl étant substitué par des groupes nitro, amino ou acylamino, sous réserve que Z soit un benzhydryle substitué par un halogène lorsque $R_1$ est amino,

sont également décrits dans la demande EP 113 226. L'activité anti-allergique de ces composés s'accompagne d'une activité sédative.

On connaît également des dérivés de benzhydryloxyéthylpipérazine de formule :

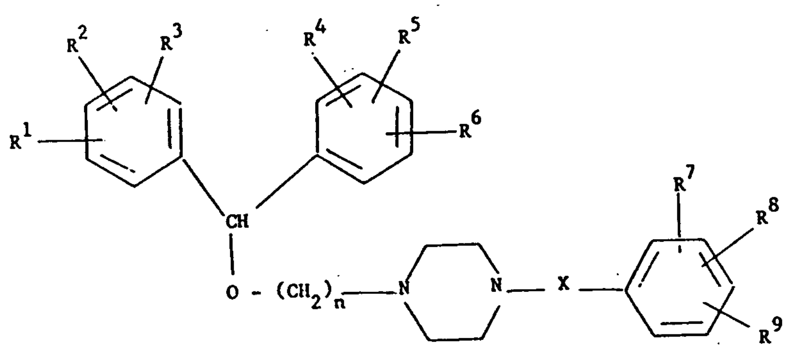

dans laquelle $R^1$ à $R^9$, identiques ou différents, représentent un atome d'hydrogène ou d'halogène ou un radical alkyle inférieur ou alkoxy inférieur, n est 2 ou 3 et X représente un radical $(CH_2)m$ (où m est 1, 2, 3 ou 4) ou un radical $-CH_2-CH=CH-$ dont le radical méthylène est uni au cycle pipérazine, et peuvent se présenter sous la forme de sels d'addition d'acides et de sels d'ammonium quaternaires.

Ces composés sont décrits dans la demande FR 2 374 318 et présentent des activités dopaminergique et anti-cholinergique.

On a maintenant trouvé une nouvelle famille de composés présentant une activité antihistaminique identique ou même supérieure à celle des dérivés précités, mais ne possédant pas de composante sédative.

Les composés selon l'invention sont des dérivés de la benzhydryloxyéthyl-pipérazine qui répondent à la formule générale ci-après :

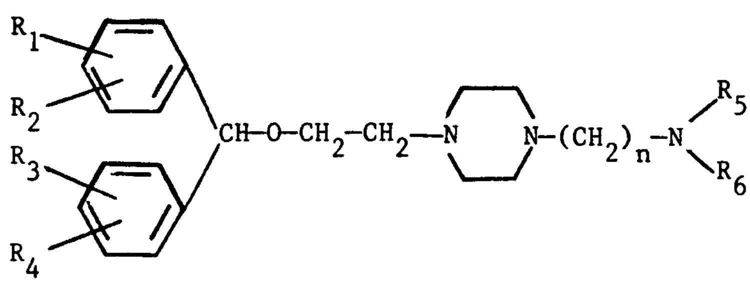

dans laquelle $R_1$, $R_2$, $R_3$ et $R_4$ indépendamment les uns des autres représentent un atome d'hydrogène, un atome d'halogène, un groupe alkyle inférieur, un groupe alcoxy inférieur ou le groupe trifluorométhyle;
n est un nombre entier compris entre 1 et 6 inclus,
$R_5$ et $R_6$ représentent, l'un un atome d'hydrogène et l'autre un groupe benzoyle substitué ou non; ou
$R_5$ et $R_6$ forment, avec l'atome d'azote auquel ils sont liés, un groupe hétérocyclique à 5 ou 6 chaînons, substitué ou non, choisi parmi les groupes ci-après : succinimidyle, 4-phénylsuccinimidyle, phtalimidyle, naphtalimidyle, phtalimidinyle, 3-hydroxyphtalimidinyle, 2-oxobenzimidazolinyle, 3-benzyl-2-oxobenzimidazolinyle, 1,2,3,4-tétrahydro-2,4-dioxoquinazolinyle, 3,4-dihydro-4-oxoquinazolinyle, 3,4-dihydro-4-oxo-2-méthylquinazolinyle, 3,7-dihydro-1,3-diméthyl-2,6-dioxo-1H-purinyle, 3,7-dihydro-3,7-diméthyl-2,6-dioxo-1H-purinyle.

Dans la présente description, on désigne par :
"alkyle inférieur" les restes d'hydrocarbures aliphatiques saturés ou insaturés contenant 1 à 6 atomes de carbone; le groupe alkyle préféré aux fins de l'invention est le groupe méthyle;
"alcoxy inférieur" un groupe hydroxy substitué par un alkyle inférieur tel que défini ci-dessus.

Le groupe benzoyle peut être, aux fins de l'invention, substitué par un groupe hydroxy, carboxy ou nitro, de préférence en position 2.

Les composés de l'invention peuvent être obtenus par réaction d'un dérivé de formule III :

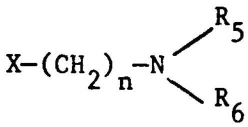

III

dans laquelle n, $R_5$ et $R_6$ sont tels que définis ci-dessus et X est un groupe halogéno ou tosyle, avec un excès de benzhydryloxyéthylpipérazine de formule IV :

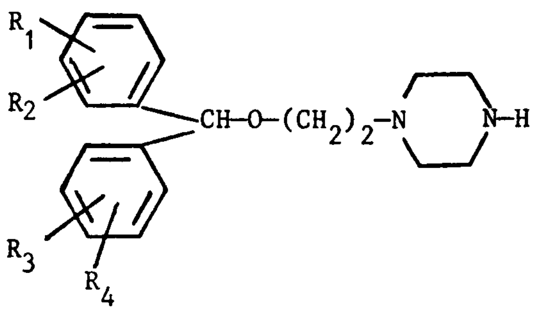

IV

dans laquelle $R_1$, $R_2$, $R_3$ et $R_4$ sont tels que définis ci-dessus, éventuellement en présence d'iodure de potassium ou de sodium.

Cette réaction est réalisée par chauffage à reflux dans un solvant approprié, tel que par exemple le toluène, le xylène ou la méthyléthylcétone.

Ce procédé permet l'obtention des composés de formule II dans lesquels $NR_5R_6$ représente notamment l'un des groupes ci-après : succinimidyle, 4-phénylsuccinimidyle, phtalimidyle, naphtalimidyle, 2-oxobenzimidazolinyle,3-benzyl-2-oxobensimidazolinyle, 3,4-dihydro-4-oxoquinazolinyle, 3,4-dihydro-2-méthyl-4-oxoquinazolinyle, 3,7-dihydro-1,3-diméthyl-2,6-dioxo-1H-purinyle, 3,7-dihydro-3,7-diméthyl-2,6-dioxo-1H-purinyle, 1,2,3,4-tétrahydro-2,4-dioxoquinazolinyle.

Les composés de formule II dans laquelle $-NR_5R_6$ représente le groupe phtalimidinyle peuvent être obtenus par réduction du composé de formule V ci-après :

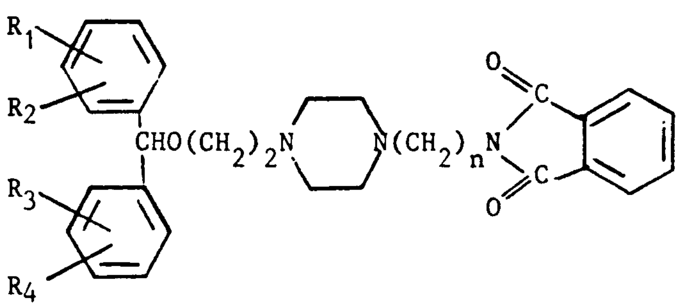

V

obtenu selon le procédé ci-dessus, qui est un composé de formule II dans laquelle $NR_5R_6$ est le groupe phtalimidyle. La réduction est avantageusement réalisée dans de l'acide acétique en présence de zinc à la température de reflux.

Les composés de formule II dans laquelle $NR_5R_6$ est le groupe 3-hydroxyphtalimidinyle peuvent avantageusement être obtenus par réaction d'un composé de formule V ci-dessus avec du borohydrure de sodium dans des solvants appropriés, tels que par exemple le méthanol, l'éthanol ou l'isopropanol.

Les composés de formule II dans laquelle $R_5$ est un atome d'hydrogène et $R_6$ un groupe 3-carboxybenzoyle peuvent être préparés à partir des composés de formule V ci-dessus, dissous dans un solvant tel que le tétrahydrofuranne (THF), par traitement à l'aide d'une solution aqueuse de sulfure de sodium à une température inférieure à 5 ° C.

Enfin, les composés de formule II dans lesquels $R_5$ représente un atome d'hydrogène et $R_6$ est un groupe benzoyle, éventuellement substitué par un groupe hydroxy ou un groupe nitro, peuvent être obtenus en faisant réagir un chlorure d'acide de formule VI :

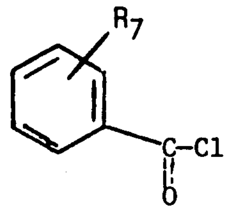

VI

dans laquelle $R_7$ est l'hydrogène, un groupe hydroxy ou un groupe nitro avec une amine de formule VII :

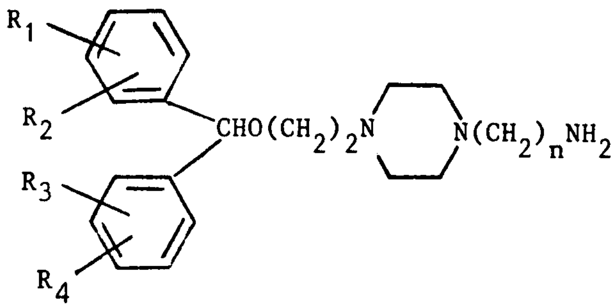

VII

dans laquelle $R_1$, $R_2$, $R_3$ et $R_4$ sont tels que définis précédemment en présence de pyridine, dans un solvant inerte, tel qu'un hydrocarbure aromatique, par exemple le benzène ou le toluène ou qu'un solvant chloré, par exemple le chloroforme ou le chlorure de méthylène.

Les sels d'addition d'acides des dérivés selon l'invention peuvent être obtenus par des procédés classiques avec des acides couramment utilisés pour obtenir des sels pharmaceutiquement acceptables, tels que par exemple l'acide acétique, l'acide chlorhydrique, l'acide trifluoroacétique, l'acide méthanesulfonique.

Les composés selon l'invention présentent des propriétés pharmacologiques intéressantes et notamment des propriétés antihistaminiques et antispasmodiques et conviennent en particulier pour le traitement des états spasmodiques et des allergies.

L'invention a donc également pour objet les compositions pharmaceutiques contenant à titre de principe actif un dérivé selon l'invention en combinaison avec un véhicule pharmaceutiquement acceptable. Les compositions selon l'invention peuvent être des compositions administrables par voie orale ou par voie injectable. Elles peuvent se présenter sous la forme de solutions, de comprimés, de pillules, de gélules ou des compositions injectables.

L'invention va être maintenant décrite plus en détail par les exemples illustratifs ci-après :

Les dérivés préparés ont été identifiés et caractérisés grâce à l'étude de leurs spectres de RMN et d'infrarouge ainsi qu'à leur analyse élémentaire.

Exemple 1 : Diméthanesulfonate de 1[2-(benzhydryloxy)éthyl]-4-[(phtalimido)-méthyl]-pipérazine (1)

Dans un réacteur on a placé 7,35 g de phtalimide, 14,8 g de benzhydryloxy-éthyl-pipérazine dans 70 ml d'éthanol absolu. On a additionné, à 0 ° C goutte à goutte, 5 ml d'une solution à 35% de formaldéhyde. Le mélange réactionnel a été agité 10 minutes à cette température. Le précipité a été essoré et recristallisé dans 50 ml d'éthanol (F = 124 ° C).

Aux 9 g du produit obtenu, dissous dans 50 ml d'acétone, on a additionné 3,7 g d'acide méthanesulfonique. Le solide a été essoré et on a obtenu 12,2 g de diméthanesulfonate (F = 116 ° C), de formule brute : $C_{28}H_{29}N_3O_3,2(CH_4O_3S)$.

| Analyse élémentaire : | | | | |
|---|---|---|---|---|
| % calculé : | C : 55,64 | H : 5,72 | N : 6,49 | S : 9,89 |
| % trouvé : | C : 55,56 | H : 5,48 | N : 6,48 | S : 9,82 |

Spectre de RMN (base, $CDCl_3$, TMS référence interne) :

7,5 ppm (m), 4H (Phtalimido); 7,0 ppm (s), 10H ($\phi_2C$); 5,1 ppm (s) 1H (CH-O); 4,4 ppm (s), 2H ($N-CH_2-N$); 3,4 ppm (t), 2H ($CH_2-O$); 2,5 ppm (m), 10H ($CH_2-N$)

Spectre IR (1% dans le KBr) :

1780 $cm^{-1}$ (C=O Asym.), 1710 $cm^{-1}$ (C=O Sym.), 1190 $cm^{-1}$ ($SO_2$) 1050 $cm^{-1}$ ($SO_2$)

Exemple 2 : Diméthanesulfonate de 1-[2-(benzhydryloxy)éthyl]-4-[2-(phtalimido)éthyl]-pipérazine (2)

Dans un réacteur on a placé 15,3 g de bromoéthylphtalimide, 36 g de benzhydryloxyéthyl-pipérazine, 500 mg d'iodure de potassium dans 250 ml de toluène anhydre. Le mélange réactionnel a été chauffé pendant 6 heures à 120 ° C. On a repris le résidu avec 100 ml d'eau. On a extrait à nouveau avec 100 ml de toluène. On a séché et on a évaporé les solvants. On a obtenu ainsi 27 g d'huile qui a cristallisé dans l'éthanol. Le solide a été essoré et on a recueilli 25,5 g de cristaux (F = 101 ° C).

A ces 25,5 g de solide, dissous dans 100 ml d'acétone, on a additionné 10,5 g d'acide méthanesulfonique. On a essoré et on a obtenu ainsi 34 g de diméthanesulfonate (F = 124 ° C), de formule brute : $C_{29}H_{31}N_3O_3$ , $2(CH_4O_3S)$.

| Analyse élémentaire : | | | | |
|---|---|---|---|---|
| % calculé :<br>% trouvé : | C : 59,26<br>C : 59,24 | H : 5,94<br>H : 5,99 | N : 6,35<br>N : 6,36 | S : 9,69<br>S : 9,79 |

Spectre RMN (base en solution dans $CDCl_3$, référence TMS) :

7,6 ppm (m) , 4H, (phtalimido); 7,1 ppm (m), 10H, ($\phi_2C$); 5,3 ppm (s) , 1H, (CH-O); 3,8 ppm (t), 2H, ($CH_2$-N-C=O); 3,5 ppm (t) , 2H, ($CH_2$-O); 2,5 ppm (m), 12H, ($CH_2$-N).

Spectre IR (1% dans le KBr)

1780 $cm^{-1}$ (C=O Asym.); 1710 $cm^{-1}$(C=O Sym.); 1200 $cm^{-1}$ ($SO_2$); 1070 $cm^{-1}$ ($SO_2$).

Exemple 3 : Diméthanesulfonate de 1-[2-(benzhydryloxy)-éthyl]-4-[2-(phtalimidino)éthyl]-pipérazine (22)

Dans un réacteur, on a placé 12 g de 1-[2-(benzhydryloxy)-éthyl]-4-[2-(phtalimido)éthyl]-pipérazine, 8,4 g de zinc dans 45 ml d'acide acétique glacial. On a chauffé à reflux le mélange pendant 5 heures. On a repris avec 100 ml d'acide chlorhydrique 2 N et 100 ml de benzène. Après décantation, on a alcalinisé la phase aqueuse par $NaHCO_3$, en présence de 100 ml de chlorure de méthylène. On a séché et on a évaporé les solvants. On a obtenu ainsi 7,4 g de solide, qui a recristallisé dans l'éthanol (F : 107 ° C).

Le diméthanesulfonate a été préparé dans l'acétone, en faisant réagir le solide avec 3,2 g d'acide méthanesulfonique. On a obtenu 10,3 g de diméthanesulfonate (F = 135 ° C) et de formule développée : $C_{29}H_{33}N_3O_2$, $2(CH_4O_3S)$.

| Analyse élémentaire : | | | | |
|---|---|---|---|---|
| % calculé :<br>% trouvé : | C : 57,50<br>C : 57,61 | H : 6,34<br>H : 6,48 | N : 6,41<br>N : 5,53 | S : 9,89<br>S : 9,95 |

Spectre RMN (base en solution dans $CDCl_3$, référence TMS) :

7,5 ppm (m), 1H, (phtalimido); 7,1 ppm (s), 10H, ($\phi_2C$); 6,2 à 7,4 ppm (m), 3H, (phtalimido); 5,2 ppm (s), 1H, (CHO) 4,3 ppm (s), 2H, ($N-CH_2$ -$\phi$); 3,6 ppm (t), 2H, ($CH_2$N-C=O); 3,4 ppm (t), 2H, ($CH_2$-O); 2,6 ppm (m), 12H, ($CH_2$-N).

Spectre IR (1% dans KBr) :

1665 $cm^{-1}$ (C=O); 1230 $cm^{-1}$ ($SO_2$); 1070 $cm^{-1}$ ($SO_2$)

Exemple 4 : Diméthanesulfonate de 1-[2-(benzhydryloxy)éthyl]-4-[(3-hydroxyphtalimidino)-éthyl]-pipérazine (23).

Dans un réacteur on a placé 15 g de 1-[2-(benzhydryloxy)-éthyl]-4-[2-(phtalimido)-éthyl]pipérazine dans 100 ml de méthanol à 90°C. On a additionné goutte à goutte, en 30 minutes, à 30°C, une solution de 4,8 g de borohydrure de sodium dans 100 ml de méthanol. Le mélange a été agité 7 heures à 30°C, puis 10 heures à température ambiante. On a filtré la solution et on a évaporé les solvants. L'huile a été reprise par 100 ml de benzène. On a additionné 50 ml d'acide chlorhydrique 1N, et le précipité formé a été filtré. La phase aqueuse a été alcalinisée par $NaHCO_3$ en présence de chlorure de méthylène (100 ml). On a séché et on a évaporé les solvants. On a recueilli 6,4 g d'huile incolore.

On a préparé le diméthanesulfonate en faisant réagir le produit obtenu, dissous dans l'éther, avec de l'acide méthanesulfonique. On a obtenu un solide de point de fusion F = 98°C et de formule brute : $C_{29}H_{33}N_3O_3$,2($CH_4O_3S$).

| Analyse élémentaire : | | | | |
|---|---|---|---|---|
| % calculé : | C : 56,10 | H : 6,18 | N : 6,33 | S : 9,65 |
| % trouvé : | C : 56,09 | H : 6,15 | N : 6,28 | S : 9,52 |

Spectre RMN (base en solution dans $CDCl_3$, référence TMS) :

6,9 à 7,6 ppm (m), 14H, (aromatiques); 5,4 ppm (s), 1H, (CHOH), 5,1 ppm (s), 1H, (CH-O); 4,5 ppm (s), 1H, (OH); 3,8 ppm (t); 2H, ($CH_2$-N-CO); 3,3 ppm (t), 2H, ($CH_2O$); 2,5 ppm (m), 12H, ($CH_2N$).

Spectre IR (1% dans KBr) :

3240 $cm^{-1}$ (OH), 1680 $cm^{-1}$ (C=O), 1200 $cm^{-1}$ ($SO_2$), 1060 $cm^{-1}$ ($SO_2$).

Exemple 5 : 1-[2-(benzhydryloxy)-éthyl]-4-[(2-carboxybenzamido)-éthyl]-pipérazine (48).

Dans un réacteur, on a placé 20 g de 1-[2-(benzhydryloxy)-éthyl]-4-[2-phtalimido)-éthyl]-pipérazine dans 200 ml de tétrahydrofuranne. On a additionné goutte à goutte, à 0°C, une solution de 20,4 g de $Na_2S$ $9H_2O$ dans 80 ml d'eau. On a décanté et on a évaporé les solvants. Le solide a été repris dans 50 ml d'eau et 100 ml de chlorure de méthylène. Après refroidissement de la solution, le pH a été ajusté à 3,7 et on a extrait par 2 fois 50 ml de chlorure de méthylène. On a séché et on a évaporé les solvants. On a obtenu 17 g de solide de point de fusion F = 45°C et de formule brute : $C_{29}H_{33}N_3O_4$.

| Analyse élémentaire : | | | |
|---|---|---|---|
| % calculé : | C : 71,46 | H : 6,78 | N : 8,62 |
| % trouvé : | C : 71,42 | H : 6,74 | N : 8,70 |

Spectre RMN (solvant : $CDCl_3$, référence interne TMS) :

10 ppm (s), 1H, (COOH); 7,8 ppm (s), 1H, (NH); 6,7 à 7,3 ppm (m), 14H, (∅); 5,0 ppm (s), 1H, (CH-O); 2,2 à 3,7 ppm (m) 16H, ($CH_2$-N).

Spectre IR (1% dans KBr) :

3200 $cm^{-1}$ (OH), 1650 $cm^{-1}$ (C=O).

Exemples 6 à 38

En répétant l'un des modes opératoires des exemples 1 à 5 on a obtenu les composés figurant dans le tableau ci-après, dans lequel apparaissent également les composés des exemples 1 à 5 ci-dessus.

TABLEAU I

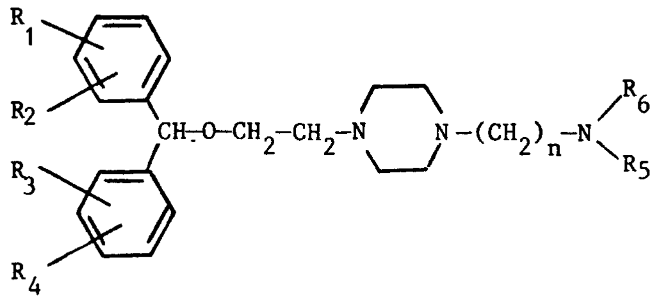

| N° | $R^1$ | $R^2$ | $R^3$ | $R^4$ | n | $NR^5R^6$ | Formule brute | Sel | Point de fusion °C | Préparé selon l'exemple n° |
|---|---|---|---|---|---|---|---|---|---|---|
| 1 | H | H | H | H | 1 | 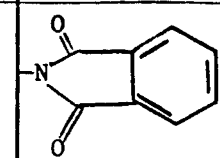 | $C_{28}H_{29}N_3O_3$ | $2xCH_3SO_3H$ | 116 | 1 |
| 2 | H | H | H | H | 2 | | $C_{29}H_{31}N_3O_3$ | " | 124 | 2 |
| 3 | H | H | H | H | 2 | 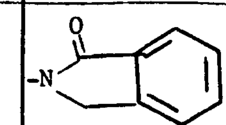 | $C_{29}H_{33}N_3O_2$ | " | 135 | 3 |
| 4 | H | H | H | H | 2 | 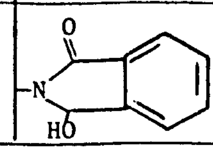 | $C_{29}H_{33}N_3O_3$ | " | 98 | 4 |

TABLEAU I (Suite)

| N° | $R^1$ | $R^2$ | $R^3$ | $R^4$ | n | $NR^5R^6$ | Formule brute | Sel | Point de fusion °C | Préparé selon l'exemple n° |
|---|---|---|---|---|---|---|---|---|---|---|
| 5 | H | H | H | H | 2 | 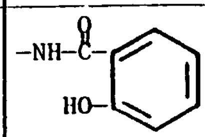 | $C_{28}H_{33}N_3O_3$ | $2xCH_3SO_3H$ | | 2 |
| 6 | 4-F | H | H | H | 2 | 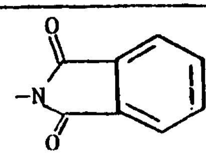 | $C_{29}H_{30}FN_3O_3$ | " | 133 | 2 |
| 7 | 4-Cl | H | H | H | 2 | | $C_{29}H_{30}ClN_3O_3$ | " | 141 | 2 |
| 8 | $4-CH_3O$ | H | H | H | 2 | | $C_{30}H_{33}N_3O_4$ | " | | 2 |
| 9 | $4-CH_3O$ | H | H | H | 2 | | $C_{30}H_{33}N_3O_4$ | $2C_4H_4O_4$ | 179 | 2 |
| 10 | 2-Cl | H | H | H | 2 | | $C_{29}H_{29}ClN_3O_3$ | $2xCH_3SO_3H$ | 94 | 2 |
| 11 | $3-CF_3$ | H | H | H | 2 | | $C_{30}H_{30}F_3N_3O_3$ | " | | 2 |
| 12 | $4-CH_3$ | H | H | H | 2 | | $C_{30}H_{33}N_3O_3$ | " | 143 | 2 |
| 13 | $3-CH_3O$ | H | H | H | 2 | | $C_{30}H_{33}N_3O_4$ | " | 150 | 2 |
| 14 | 3-Cl | H | H | H | 2 | | $C_{29}H_{30}ClN_3O_3$ | " | 156 | 2 |
| 15 | 4-Cl | H | 4-Cl | H | 2 | | $C_{29}H_{29}Cl_2N_3O_3$ | " | 190 | 2 |

TABLEAU I (Suite)

| N° | $R^1$ | $R^2$ | $R^3$ | $R^4$ | n | $NR^5R^6$ | Formule brute | Sel | Point de fusion °C | Préparé selon l'exemple n° |
|---|---|---|---|---|---|---|---|---|---|---|
| 16 | 2-Cl | 6-Cl | H | H | 2 | 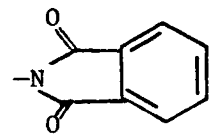 | $C_{29}H_{29}Cl_2N_3O_3$ | $2xCH_3SO_3H$ | 175 | 2 |
| 17 | $2-CF_3$ | H | $2-CF_3$ | H | 2 | | $C_{31}H_{29}F_6N_3O_3$ | " | | 2 |
| 18 | H | H | H | H | 3 | | $C_{30}H_{33}N_3O_3$ | " | 144 | 2 |
| 19 | H | H | H | H | 4 | | $C_{31}H_{35}N_3O_3$ | " | | 2 |
| 20 | H | H | H | H | 5 | | $C_{32}H_{37}N_3O_3$ | " | | 2 |
| 21 | H | H | H | H | 6 | | $C_{33}H_{39}N_3O_3$ | " | | 2 |
| 22 | H | H | H | H | 2 | 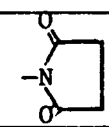 | $C_{25}H_{31}N_3O_3$ | " | 105 | 2 |
| 23 | H | H | H | H | 2 | 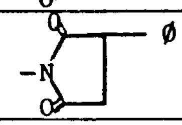 | $C_{31}H_{35}N_3O_3$ | " | 110 | 2 |
| 24 | H | H | H | H | 2 | 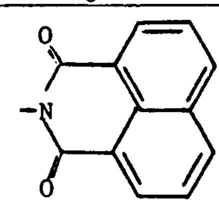 | $C_{33}H_{33}N_3O_3$ | " | >200 | 2 |

TABLEAU I (Suite)

| N° | $R^1$ | $R^2$ | $R^3$ | $R^4$ | n | $NR^5R^6$ | Formule brute | Sel | Point de fusion °C | Préparé selon l'exemple n° |
|---|---|---|---|---|---|---|---|---|---|---|
| 25 | H | H | H | H | 2 | 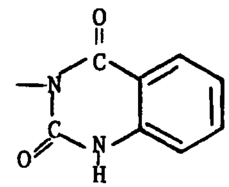 | $C_{29}H_{32}N_4O_3$ | $2CH_3SO_3H$ | 194 | 2 |
| 26 | H | H | H | H | 2 | 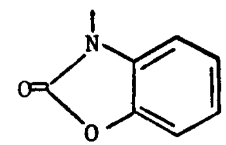 | $C_{29}H_{33}N_3O_3$ | " | 172 | 2 |
| 27 | H | H | H | H | 2 | 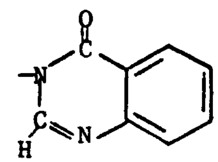 | $C_{29}H_{32}N_4O_2$ | " | | 2 |
| 28 | H | H | H | H | 2 | 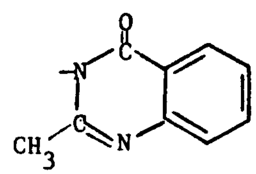 | $C_{30}H_{34}N_4O_2$ | " | | 2 |

TABLEAU I (Suite)

| N° | $R^1$ | $R^2$ | $R^3$ | $R^4$ | n | $NR^5R^6$ | Formule brute | Sel | Point de fusion °C | Préparé selon l'exemple n° |
|---|---|---|---|---|---|---|---|---|---|---|
| 29 | H | H | H | H | 2 | 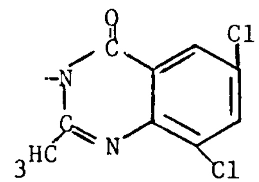 | $C_{30}H_{32}Cl_2N_4O_2$ | $2CH_3SO_3H$ | 148 | 2 |
| 30 | H | H | H | H | 2 | 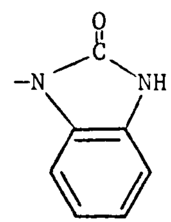 | $C_{28}H_{32}N_4O_2$ | " | | 2 |
| 31 | H | H | H | H | 3 | | $C_{29}H_{34}N_4O_2$ | " | | 2 |
| 32 | H | H | H | H | 2 | 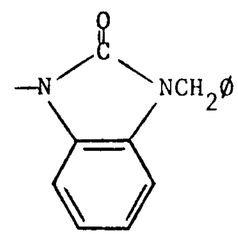 | $C_{35}H_{38}N_4O_2$ | " | 173 | 2 |
| 33 | H | H | H | H | 2 | Théophylline | $C_{28}H_{34}N_6O_3$ | " | | 2 |
| 34 | H | H | H | H | 4 | | $C_{30}H_{38}N_6O_3$ | " | | 2 |

TABLEAU I (Suite)

| N° | $R^1$ | $R^2$ | $R^3$ | $R^4$ | n | $NR^5R^6$ | Formule brute | sel | Point de fusion °c | Préparé selon l'exemple n° |
|---|---|---|---|---|---|---|---|---|---|---|
| 35 | 4-F | H | H | H | 2 | Théophylline | $C_{28}H_{33}FN_6O_3$ | $2CH_3SO_3H$ | 156 | 2 |
| 36 | 4-$CH_3$ | H | H | H | 2 | | $C_{29}H_{36}N_6O_3$ | " | 160 | 2 |
| 37 | 4-Cl | H | H | H | 2 | | $C_{28}H_{33}N_6ClO_3$ | " | 159 | 2 |
| 38 | 4-Cl | H | 4-Cl | H | 2 | | $C_{28}H_{32}Cl_2N_6O_3$ | " | 175 | 2 |
| 39 | 4-$CH_3O$ | H | H | H | 2 | | $C_{29}H_{36}N_6O_4$ | " | 154 | 2 |
| 40 | 2-Cl | H | H | H | 2 | | $C_{28}H_{33}ClN_6O_3$ | " | 154 | 2 |
| 41 | 4-$CH_3$ | H | H | H | 4 | | $C_{31}H_{40}N_6O_3$ | " | | 2 |
| 42 | 4-F | H | H | H | 4 | | $C_{30}H_{37}FN_6O_3$ | " | | 2 |
| 43 | 4-Cl | H | H | H | 4 | | $C_{30}H_{37}ClN_6O_3$ | " | 172 | 2 |
| 44 | 4-F | H | 4-F | H | 2 | | $C_{28}H_{32}F_2N_6O_3$ | " | 164 | 2 |
| 45 | 4-Cl | H | 4-Cl | H | 4 | | $C_{30}H_{36}Cl_2N_6O_3$ | " | | 2 |
| 46 | 4-$CH_3O$ | H | H | H | 4 | | $C_{31}H_{40}N_6O_4$ | " | | 2 |

TABLEAU I (Suite)

| N° | $R^1$ | $R^2$ | $R^3$ | $R^4$ | n | $NR^5R^6$ | Formule brute | Sel | Point de fusion °C | Préparé selon l'exemple n° |
|---|---|---|---|---|---|---|---|---|---|---|
| 47 | H | H | H | H | 2 | Théobromine | $C_{28}H_{34}N_6O_3$ | $2CH_3SO_3H$ | | 2 |
| 48 | H | H | H | H | 2 | 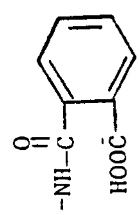 | $C_{29}H_{33}N_3O_4$ | base | 45 | 5 |
| 49 | H | H | H | H | 2 | 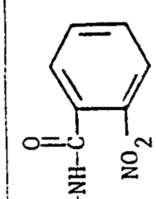 | $C_{28}H_{32}N_4O_4$ | $2CH_3SO_3H$ | 170 | 2 |

La toxicité des composés de l'invention a été déterminée selon le mode opératoire ci-après :

I - ESSAI DE TOXICITE

Détermination de la dose létale cinquante ($D.L._{50}$) chez la souris.

Les dérivés étudiés ont été administrés par voie intrapéritonéale à des groupes composés de cinq souris mâles et cinq souris femelles, à raison de 0,1 ml pour dix grammes de poids corporel. La dose létale 50 à été évaluée à partir de la mortalité observée.

Les résultats obtenus sont reproduits dans le tableau II ci-après :

TABLEAU II

| Numéro dérivé | $D.L._{50}$ $mg.kg^{-1}$ I.P. |
|---|---|
| 2 | 211 |
| 19 | 133 |
| 22 | 146 |
| 32 | 400 |
| 33 | 240 |
| 5 | 185 |
| 36 | 178 |
| 49 | 131 |

II - ESSAIS PHARMACOLOGIQUES

Les propriétés pharmacologiques des composés de l'invention ont été déterminées en utilisant les tests ci-après :

Protocoles d'essais

A - Etude de la motilité spotanée

L'activité motrice des souris a été déterminée à l'aide de l'actimètre photo-électrique de Boissier et Simon

Les souris sont placées par cinq dans une boîte fermée par un couvercle, et traversée par deux rayons lumineux perpendiculaires que les souris coupent en se déplaçant.

Ces déplacements sont comptabilisés par un compteur relevé après trente minutes et une heure.

B - Comportement d'exploration

Trente minutes après l'administration intrapéritonéale des dérivés selon l'invention, chaque souris est placée sur une planche à trous automatisée pendant cinq minutes, et on note,minute par minute, le nombre de trous explorés.

Une dose efficace 50 peut être calculée en fonction des résultats obtenus.

C - Action myorelaxante (test de traction)

Ce test apprécie la présence ou l'absence de redressements d'une souris présentée par ses pattes antérieures à un fil métallique horizontal.

On note le nombre de souris qui ne peuvent accrocher au fil une de leurs pattes postérieures avant cinq secondes. Une dose efficace 50 peut être calculée en fonction des résultats obtenus.

D - Interaction avec le pentobarbital

On recherche une éventuelle augmentation du sommeil barbiturique en administrant par voie intrapéritonéale le produit à tester cinq minutes avant l'injection intrapéritonéale du pentobarbital.

Une dose efficace 50 peut être calculée en fonction des résultats obtenus.

E - Activité analgésique périphérique

Une douleur péritonéale de la souris est provoquée par injection intrapéritonéale de phénylbenzoquinone (PBQ). On recherche la diminution du syndrome douloureux caractérisé par une torsion abdominale à l'aide d'une injection du produit à essayer trente minutes avant l'administration de la P.B.Q.

La dose efficace 50 est calculée en fonction du pourcentage de diminution du syndrome douloureux par rapport aux témoins.

F - Bronchospasme par inhalation d'une solution d'histamine

Les cobayes sont placés dans une enceinte fermée où l'histamine est aérolisée, et seuls sont retenus ceux qui montrent des signes très nets d'asphyxie dans les quatre minutes.

La substance à étudier est administrée à des lots de cobayes trente minutes avant un nouveau passage dans l'enceinte pour contrôler la résistance à l'histamine. Un cobaye est considéré comme protégé s'il résiste pendant dix minutes à l'aérosol d'histamine sans montrer de signe d'asphyxie.

Une dose efficace 50 est calculée en fonction des résultats obtenus.

G - Activité antihistaminique.

Recherche de la dose protectrice chez cinquante pour cent des cobayes vis-à-vis d'une dose mortelle d'histamine.

Administration du produit trente minutes avant l'injection intraveineuse de chlorhydrate d'histamine. La dose efficace 50 est calculée en fonction des résultas obtenus.

Résultats.

Les résultats obtenus sont consignés dans le tableau III ci-après.

Essais comparatifs.

A titre de comparaison, on a effectué les tests ci-dessus avec la Terfenadine, comme composé de l'art antérieur ; ce composé a donné les résultats ci-après dans les différents tests définis ci-dessus.

SOURIS.

| | |
|---|---|
| Toxicité par voie intrapéritonéale | $D.L._{50}$ = 100 $mg.kg^{-1}$ I.P. |
| Activité motrice | Diminution importante à 25 $mg.kg^{-1}$ I.P. |
| Comportement d'exploration | $D.E._{50}$ = 37 $mg.kg^{-1}$ I.P. |
| Action myorelaxante - Test de traction | $D.E._{50}$ = 31 $mg.kg^{-1}$ I.P. |
| Interaction avec le pentobarbital | $D.E._{50}$ = 25 $mg.kg^{-1}$ I.P. |
| Activité analgésique périphérique | $D.E._{50}$ = 3,1 $mg.kg^{-1}$ I.P. |

COBAYE

| | |
|---|---|
| Bronchospasme par voie orale | $D.E._{50}$ = 1,60 $mg.kg^{-1}$ P.O. |
| Choc histaminique par voie orale | $D.E._{50}$ = 2,58 $mg.kg^{-1}$ P.O. |

Les résultats obtenus avec les composés de l'invention figurent dans le tableau III.

Ces résultats montrent que, par rapport au composé de l'art antérieur testé, les composés de l'invention présentent une activité antihistaminique sensiblement équivalente ou supérieure, mais ne possèdent pas de composante sédative.

TABLEAU III Résultats d'essais pharmacologiques

| Numéro Dérivé | Activité motrice (souris) $mg.kg^{-1}$ I.P. | Comportement d'exploration (souris) $mg.kg^{-1}$ I.P. | Action myorelaxante Test de traction (souris) $mg.kg^{-1}$ I.P. | Interaction avec le Pentobarbital (souris) $mg.kg^{-1}$ I.P. | Activité analgésique périphérique (souris) $DE_{50} mg.kg^{-1}$ | Bronchospasme (cobaye) $DE_{50} mg.kg^{-1}$ | Choc histaminique (cobaye) $DE_{50} mg.kg^{-1}$ |
|---|---|---|---|---|---|---|---|
| Composés de l'invention | | | | | | | |
| 2 | aucune modification à 50 | aucune modification à 50 | aucune modification à 100 | aucune interaction à 25 | 3,12 (I.P.) | 3,67 (I.P.) | 2,03 (I.P.) |
| 19 | aucune modification à 25 | aucune modification à 25 | aucune modification à 25 | aucune interaction à 25 | 1,56 (I.P.) | 5,34 (P.O.) | 2,20 (P.O.) |
| 22 | aucune modification à 25 | aucune modification à 12,5 | aucune modification à 25 | aucune interaction à 25 | 35 (P.O.) | 3,96 (I.P.) | 3,25 (I.P.) |
| 32 | aucune modification à 25 | aucune modification à 25 | aucune modification à 25 | aucune interaction à 6,25 | - | 33% à 12,5 (P.O.) | 2,30 (P.O.) |
| 33 | aucune modification à 12,5 | aucune modification à 25 | aucune modification à 25 | aucune interaction à 12,5 | 0 | 0,59 (P.O.) | 2,57 (P.O.) |
| 5 | augmentation de l'activité | aucune modification à 25 | aucune modification à 25 | aucune interaction à 25 | 6,25 (S.C.) | 2,06 (I.P.) | 5,24 (P.O.) |
| 36 | augmentation à dose faible -aucune modification à dose forte | aucune modification à 25 | aucune modification à 25 | aucune modification à 25 | 1,56 (S.C.) | 0,19 (P.O.) | 0,55 (P.O.) |

**TABLEAU III** Résultats d'essais pharmacologiques (Suite)

| Numéro Dérivé | Activité motrice (souris) $mg.kg^{-1}$ I.P. | Comportement d'exploration (souris) $mg.kg^{-1}$ I.P. | Action myorelaxante Test de traction (souris) $mg.kg^{-1}$ I.P. | Interaction avec le Pentobarbital (souris) $mg.kg^{-1}$ I.P. | Activité analgésique périphérique (souris) $DE_{50} mg.kg^{-1}$ | Bronchospasme (cobaye) $DE_{50} mg.kg^{-1}$ | Choc histaminique (cobaye) $DE_{50} mg.kg^{-1}$ |
|---|---|---|---|---|---|---|---|
| 49 | augmentation de l'activité | aucune modification à 25 | | aucune interaction à 25 | 2,6 (S.C.) | 3,I25 (P.O.) | |

## Revendications

### Revendications pour les Etats contractants suivants : BE, CH, DE, FR, GB, IT, LI, LU, NL, SE

**1.** Dérivés de la benzhydryloxyéthyl-pipérazine répondant à la formule générale II :

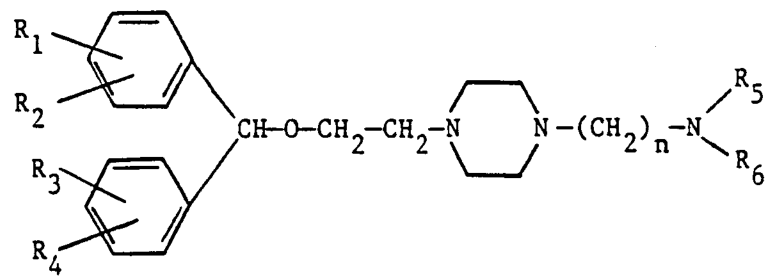

dans laquelle :

$R_1$ $R_2$, $R_3$ et $R_4$, indépendamment les uns des autres représentent un atome d'hydrogène, un atome d'halogène, un groupe alkyle inférieur, contenant 1 à 6 atomes de carbone, un groupe alcoxy inférieur contenant 1 à 6 atomes de carbone ou le groupe trifluorométhyle ;

n est un nombre entier compris entre 1 et 6 inclus ;

$R_5$ et $R_6$ représentent, l'un un atome d'hydrogène et l'autre un groupe benzoyle substitué par un groupe hydroxy, carboxy ou nitro, ou non substitué ; ou

$R_5$ et $R_6$ forment, avec l'atome d'azote auquel ils sont liés, un groupe hétérocyclique à 5 ou 6 chaînons, substitué ou non, choisi parmi les groupes ci-après : succinimidyle, 4-phénylsuccinimidyle, phtalimidyle, naphtalimidyle, phtalimidinyle, 3-hydroxyphtalimidinyle, 2-oxobenzimidazolinyle, 3-benzyl-2-oxobenzimidazolinyle, 1,2,3,4-tétrahydro-2,4-dioxoquinazolinyle, 3,4-dihydro-4-oxoquinazolinyle, 3,4-dihydro-4-oxo-2-méthylquinazolinyle, 3,7-dihydro-1,3-diméthyl-2,6-dioxo-1H-purinyle, 3,7-dihydro-3,7-diméthyl-2,6-dioxo-1H-purinyle.

**2.** Procédé pour l'obtention des dérivés selon la revendication 1, dans lesquels $NR_5R_6$ représente l'un des groupes ci-après : succinimidyle, 4-phénylsuccinimidyle, phtalimidyle, naphtalimidyle, 2-oxobenzimidazolinyle, 3-benzyl-2-oxobenzimidazolinyle 3,4-dihydro-4-oxo-quinazolinyle, 3,4-dihydro-2-méthyl-4-oxo-quinazolinyle, 3,7-dihydro-1,3-diméthyl-2,6-dioxo-1H-purinyle, 3,7-dihydro-3,7-diméthyl-,6-dioxo-1H-purinyle, ou 1, 2, 3, 4-tetrahydro-2,4-dioxoquinazolinyle caractérisé en ce qu'il consiste à faire réagir à reflux un dérive de formule III:

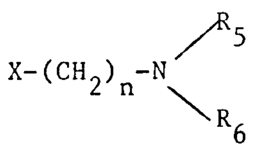

III

dans laquelle n, $R_5$ et $R_6$ sont tels que définis dans la revendication 1, et X est un groupe halogéno ou tosyle, avec un excès de benzydryloxyéthyl-pipérazine de formule IV :

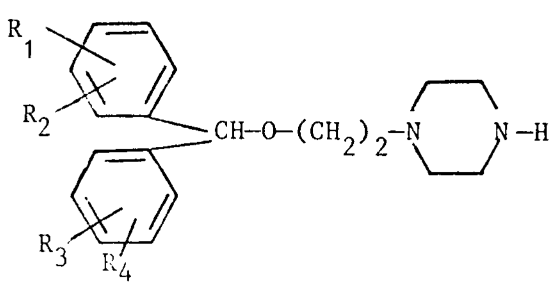

IV

dans laquelle $R_1$, $P_2$, $R_3$ et $R_4$ sont tels que définis dans la revendication 1, dans un solvant approprié tel que le toluène, le xylène ou la méthyléthylcétone.

**3.** Procédé pour l'obtention des dérivés selon la revendication 1, dans lesquels $NR_5R_6$ représente le groupe phtalimidinyle, caractérisé en ce qu'il consiste à réduire, en présence de zinc et à reflux, un composé de formule V :

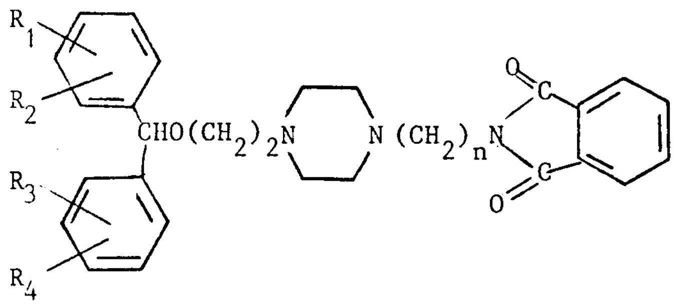

V

obtenu par le procédé selon la revendication 2.

4. Procédé pour l'obtention des dérivés selon la revendication 1, dans lesquels $NR_5R_6$ est le groupe 3-hydroxyphtalimidinyle, caractérisé en ce qu'il consiste à faire réagir un composé de formule V tel que défini dans la revendication 3 avec du borohydrure de sodium dans des solvants appropriés, tels que par exemple le méthanol, l'éthanol ou l'isopropanol.

5. Procédé pour l'obtention des dérivés selon la revendication 1, dans lesquels $R_5$ est l'hydrogène et $R_6$ est un groupe 3-carboxybenzoyle, caractérisé en ce qu'il consiste à faire réagir un composé de formule V tel que défini dans la revendication 3, avec une solution aqueuse de sulfure de sodium à une température inférieure à 5 ° C.

6. Procédé pour l'obtention des dérivés selon la revendication 1, dans lesquels $R_5$ représente un atome d'hydrogène et $R_6$ est un groupe benzoyle, éventuellement substitué par un groupe hydroxy ou un groupe nitro, caractérisé en ce qu'il consiste à faire réagir un chlorure d'acide de formule VI :

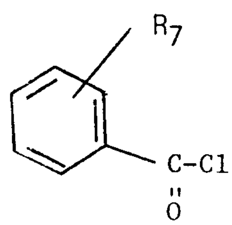

VI

dans laquelle $R_7$ est l'hydrogène, un groupe hydroxy ou un groupe nitro avec une amine de formule VII :

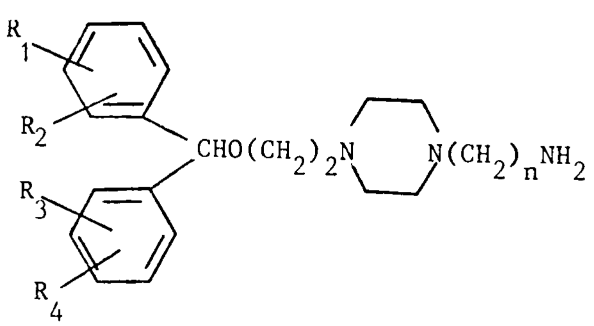

VII

dans laquelle $R_1$, $R_2$, $R_3$ et $R_4$ sont tels que définis précédemment, en présence de pyridine, dans un solvant inerte, tel qu'un hydrocarbure aromatique, par exemple le benzène ou le toluène, ou qu'un solvant chloré, par exemple le chloroforme ou le chlorure de méthylène.

7. Procédé selon l'une quelconque des revendications 2 à 6, caractérisé en ce que les dérivés obtenus sont transformés en leurs sels d'addition d'acides.

8. Compositions pharmaceutiques, caractérisées en ce qu'elles contiennent, à titre d'ingrédient actif, un dérivé selon la revendication 1, en combinaison avec un véhicule pharmaceutiquement acceptable.

**Revendications pour les Etats contractants suivants : AT, ES**

1. Procédé pour l'obtention de dérivés de la benzhydryloxyéthyl-pipérazine répondant à la formule générale II :

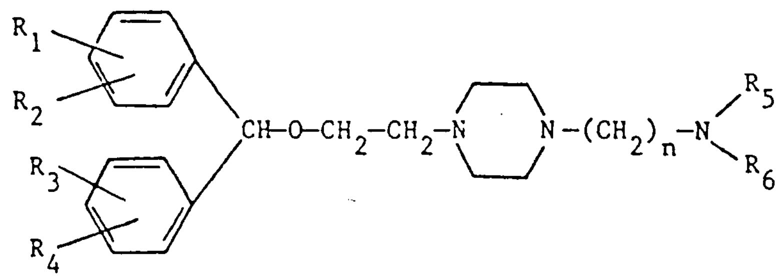

dans laquelle :
$R_1$, $R_2$, $R_3$ et $R_4$ indépendamment les uns des autres représentent un atome d'hydrogène, un atome d'halogène, un groupe alkyle inférieur contenant 1 à 6 atomes de carbone, un groupe alcoxy inférieur contenant 1 à 6 atomes de carbone ou le groupe trifluorométhyle ;
n est un nombre entier compris entre 1 et 6 inclus ;
$R_5$ et $R_6$ représentent, l'un un atome d'hydrogène et l'autre un groupe benzoyle substitué par un groupe hydroxy, carboxy ou nitro, ou non substitué; ou
$R_5$ et $R_6$ forment, avec l'atome d'azote auquel ils sont liés, un groupe hétérocyclique à 5 ou 6 chaînons, substitué ou non, choisi parmi les groupes ci-après : succinimidyle, 4-phénylsuccinimidyle, phtalimidyle, naphtalimidyle,
2-oxozimidazolinyle, 3-benzyl-2-oxobenzimidazolinyle, 1, 2, 3, 4 - tétrahydro-2,4-dioxoquinazolinyle, 3,4-dihydro-4-oxoquinazolinyle, 3,4-dihydro-4-oxo-2-méthylquinazolinyle, 3,7-dihydro-1,3-diméthyl - 2,6-dioxo-1H-purinyle, 3,7-dihydro- 3,7 -diméthyl- 2,6 -dioxo -1H - purinyle, caractérisé en ce qu'il consiste à faire réagir à reflux un dérivé de formule III :

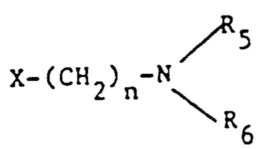

III

dans laquelle n, $R_5$ et $R_6$ sont tels que définis ci-dessus, et X est un groupe halogéno ou tosyle, avec un excès de benzhydryloxyéthylpipérazine de formule IV :

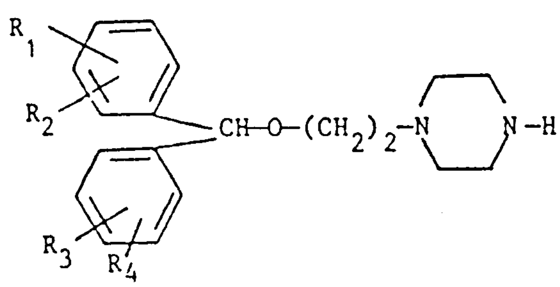

IV

dans laquelle $R_1$, $R_2$, $R_3$ et $R_4$ sont tels que définis ci-dessus, dans un solvant approprié tel que le toluène, le xylène ou la méthyléthylcétone,
et à transformer éventuellement les dérivés obtenus en leurs sels d'addition d'acides.

2. Procédé pour l'obtention de dérivés de la benzhydryloxyéthyl-pipérazine répondant à la formule

générale II :

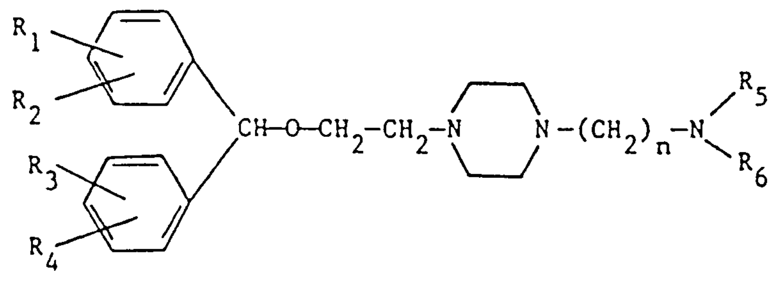

dans laquelle :
$R_1$, $R_2$, $R_3$ et $R_4$, indépendamment les uns des autres représentent un atome d'hydrogène, un atome d'halogène, un groupe alkyle inférieur contenant 1 à 6 atomes de carbone, un groupe alcoxy inférieur contenant 1 à 6 atomes de carbone ou le groupe trifluorométhyle;
n est un nombre entier compris entre 1 et 6 inclus;
$R_5$ et $R_6$ forment, avec l'azote auquel ils sont liés, le groupe phtalimidinyle, caractérisé en ce qu'il consiste à réduire, en présence de zinc et à reflux, un composé de formule V :

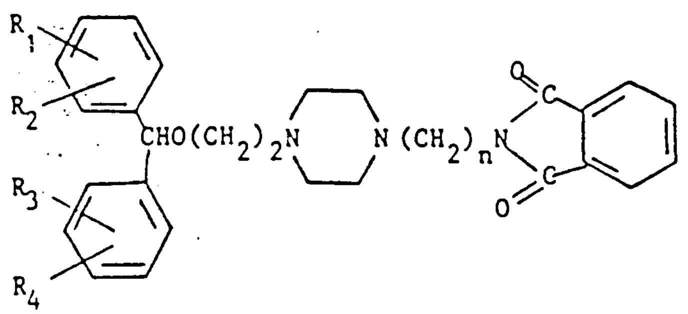

V

obtenu par le procédé selon la revendication 1,
et à transformer éventuellement les dérivés obtenus en leurs sels d'addition d'acides.

**3.** Procédé pour l'obtention de dérivés de la benzhydryioxyéthyl-pipérazine répondant à la formule générale II :

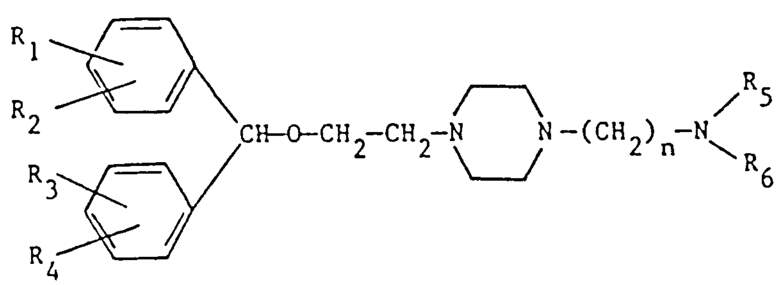

dans laquelle :
$R_1$, $R_2$, $R_3$ et $R_4$, indépendamment les uns des autres représentent un atome d'hydrogène, un atome d'halogène, un groupe alkyle inférieur contenant 1 à 6 atomes de carbone un groupe alcoxy inférieur contenant 1 à 6 atomes de carbone ou le groupe trifluorométhyle;
n est un nombre entier compris entre 1 et 6 inclus;
$R_5$ et $R_6$ forment, avec l'azote auquel ils sont liés le groupe 3-hydroxyphtalimidinyle, caractérisé en ce qu'il consiste à faire réagir un composé de formule V tel que défini dans la revendication 1 avec du borohydrure de sodium dans des solvants appropriés, tels que par exemple le méthanol, l'éthanol ou

l'isopropanol,
et à transformer éventuellement les dérivés obtenus en leurs sels d'addition d'acides.

**4.** Procédé pour l'obtention de dérivés de la benzhydryloxyéthyl-pipérazine répondant à la formule générale II :

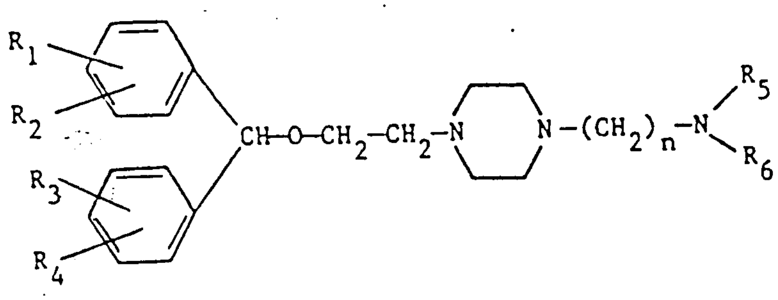

dans laquelle :
$R_1$, $R_2$, $R_3$ et $R_4$, indépendamment les uns des autres représentent un atome d'hydrogène, un atome d'halogène, un groupe alkyle inférieur contenant 1 à 6 atomes de carbone un groupe alcoxy inférieur contenant 1 à 6 atomes de carbone ou le groupe trifluorométhyle;
n est un nombre entier compris entre 1 et 6 inclus;
$R_5$ est l'hydrogène et $R_6$ est un groupe 3-carboxybenzoyle, caractérisé en ce qu'il consiste à faire réagir un composé de formule V tel que défini dans la revendication 1, avec une solution aqueuse de sulfure de sodium à une température inférieure à 5 ° C,
et à transformer éventuellement les dérivés obtenus en leurs sels d'addition d'acides.

**5.** Procédé pour l'obtention de dérivés de la benzhydryloxyéthyl-pipérazine répondant à la formule générale II :

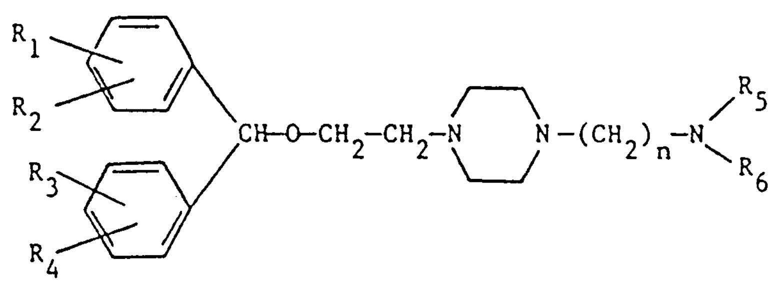

dans laquelle :
$R_1$, $R_2$, $R_3$ et $R_4$, indépendamment les uns des autres représentent un atome d'hydrogène, un atome d'halogène, un groupe alkyle inférieur contenant 1 à 6 atomes de carbone un groupe alcoxy inférieur contenant 1 à 6 atomes de carbone ou le groupe trifluorométhyle;
n est un nombre entier compris entre 1 et 6 inclus;
$R_5$ représente un atome d'hydrogène et $R_6$ est un groupe benzoyle éventuellement substitué par un groupe hydroxy ou un groupe nitro, caractérisé en ce qu'il consiste à faire réagir un chlorure d'acide de formule VI :

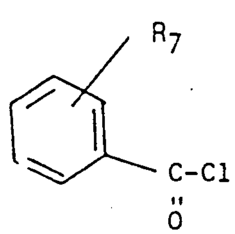

VI

dans laquelle $R_7$ est l'hydrogène, un groupe hydroxy ou un groupe nitro avec une amine de formule VII :

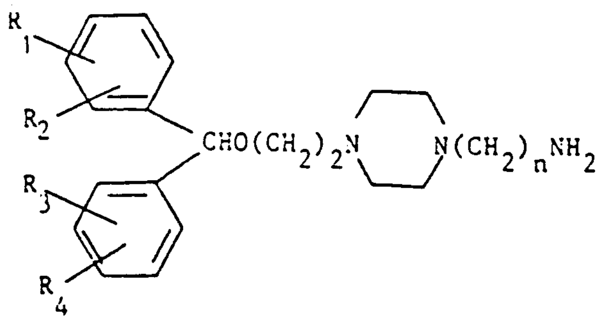

VII

dans laquelle $R_1$, $R_2$, $R_3$ et $R_4$ sont tels que définis précédemment, en présence de pyridine, dans un solvant inerte, tel qu'un hydrocarbure aromatique, par exemple le benzène ou le toluène, ou qu'un solvant chloré, par exemple le chloroforme ou le chlorure de méthylène.
et à transformer éventuellement les dérivés obtenus en leurs sels d'addition d'acides.

**6.** Utilisation des dérivés de la benzhydryloxyéthyl-pipérazine répondant à la formule générale II :

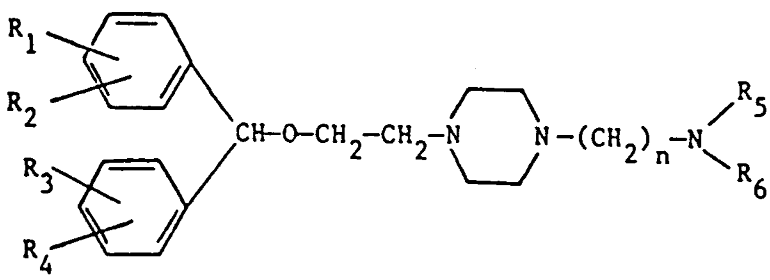

dans laquelle :
$R_1$, $R_2$, $R_3$ et $R_4$, indépendamment les uns des autres représentent un atome d'hydrogène, un atome d'halogène, un groupe alkyle inférieur contenant 1 à 6 atomes de carbone, un groupe alcoxy inférieur contenant 1 à 6 atomes de carbone ou le groupe trifluorométhyle ;
n est un nombre entier compris entre 1 et 6 inclus ;
$R_5$ et $R_6$ représentent, l'un un atome d'hydrogène et l'autre un groupe benzoyle substitué par un groupe hydroxy, carboxy ou nitro ou non substitué ; ou
$R_5$ et $R_6$ forment, avec l'atome d'azote auquel ils sont liés, un groupe hétérocyclique à 5 ou 6 chaînons, substitué ou non, choisi parmi les groupes ci-après : succinimidyle, 4-phénylsuccinimidyle, phtalimidyle, naphtalimidyle, phtalimidinyle, 3-hydroxyphtalimidinyle, 2-oxobenzimidazolinyle, 3-benzyl-2-oxo-benzimidazolinyle, 1,2,3,4-tétrahydro-2,4-dioxoquinazolinyle, 3,4-dihydro-4-oxoquinazolinyle, 3,4-dihydro-4-oxo-2-méthylquinazolinyle, 3,7-dihydro-1,3-diméthyl-2,6-dioxo-1H-purinyle, 3,7-dihydro-3,7-diméthyl-2,6-dioxo-1H-purinyle, pour la préparation des médicaments pour le traitement des états spasmodiques ou des allergies.

**Revendications pour l'Etat contractant suivant : GR**

**1.** Dérivés de la benzhydryloxyéthyl-pipérazine répondant à la formule générale II :

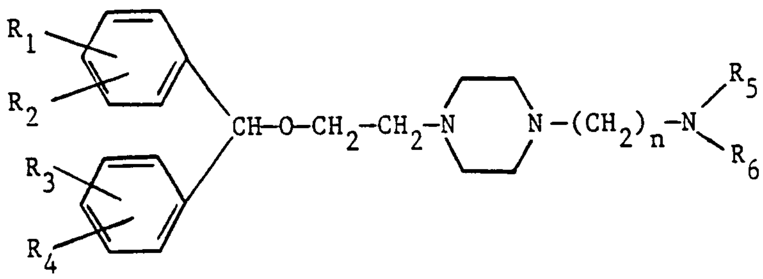

dans laquelle :

$R_1$, $R_2$, $R_3$ et $R_4$, indépendamment les uns des autres représentent un atome d'hydrogène, un atome d'halogène, un groupe alkyle inférieur, contenant 1 à 6 atomes de carbone, un groupe alcoxy inférieur contenant 1 à 6 atomes de carbone ou le groupe trifluorométhyle ;

n est un nombre entier compris entre 1 et 6 inclus ;

$R_5$ et $R_6$ représentent, l'un un atome d'hydrogène et l'autre un groupe benzoyle substitué par un groupe hydroxy, carboxy ou nitro, ou non substitué ; ou

$R_5$ et $R_6$ forment, avec l'atome d'azote auquel ils sont liés, un groupe hétérocyclique à 5 ou 6 chaînons, substitué ou non, choisi parmi les groupes ci-après : succinimidyle, 4-phénylsuccinimidyle, phtalimidyle, naphtalimidyle, phtalimidinyle, 3-hydroxyphtalimidinyle, 2-oxobenzimidazolinyle, 3-benzyl-2-oxobenzimidazolinyle, 1,2,3,4-tétrahydro-2,4-dioxoquinazolinyle, 3,4-dihydro-4-oxoquinazolinyle, 3,4-dihydro-4-oxo-2-méthylquinazolinyle, 3,7-dihydro-1,3-diméthyl-2,6-dioxo-1H-purinyle, 3,7-diflydro-3,7-diméthyl-2,6-dioxo-1H-purinyle.

**2.** Procédé pour l'obtention des dérivés selon la revendication 1, dans lesquels $NR_5R_6$ représente l'un des groupes ci-après : succinimidyle, 4-phénylsuccinimidyle, phtalimidyle, naphtalimidyle, 2-oxobenzimidazolinyle, 3-benzyl-2-oxobenzimidazolinyle, 3,4-dihydro-4-oxo-quinazolinyle, 3,4-dihydro-2-méthyl-4-oxoquinazolinyle, 3,7-dihydrol-1,3-diméthyl-2,6-dioxo-1H-purinyle, 3,7-dihydro-3,7-diméthyl-2,6-dioxo-1H-purinyle, ou 1, 2, 3, 4-tétrahydro-2,4-dioxoquinazolinyle caractérisé en ce qu'il consiste à faire réagir à reflux un dérivé formule III :

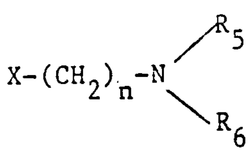

III

dans laquelle n, $R_5$ et $R_6$ sont tels que définis dans la revendication 1, et X est un groupe halogéno ou tosyle, avec un excès de benzhydryloxéthyl-pipérazine de formule IV :

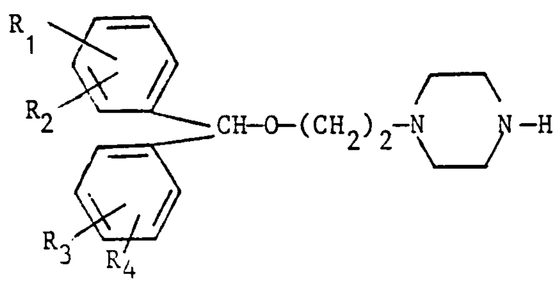

IV

dans laquelle $R_1$ $R_2$, $R_3$ et $R_4$ sont tels que définis dans la revendication 1, dans un solvant approprié tel que le toluène, le xylène ou la méthyléthylcétone.

**3.** Procédé pour l'obtention des dérivés selon la revendication 1, dans lesquels $NR_5R_6$ représente le groupe phtalimidinyle, caractérisé en ce qu'il consiste à réduire, en présence de zinc et à reflux, un composé de formule V :

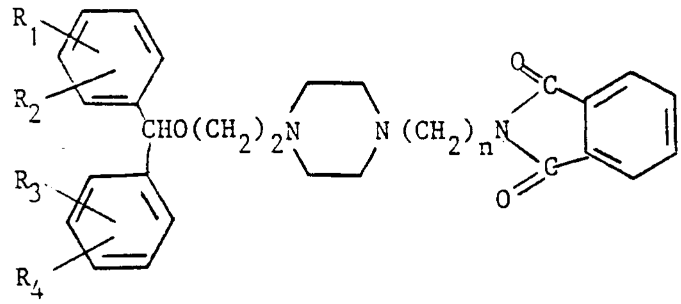

V

obtenu par le procédé selon la revendication 2.

**4.** Procédé pour l'obtention des dérivés selon la revendication 1, dans lesquels $NR_5R_6$ est le groupe 3-hydroxyphtalimidinyle, caractérisé en ce qu'il consiste à faire réagir un composé de formule V tel que défini dans la revendication 3 avec du borohydrure de sodium dans des solvants appropriés, tels que par exemple le méthanol, l'éthanol ou l'isopropanol.

**5.** Procédé pour l'obtention des dérivés selon la revendication 1, dans lesquels $R_5$ est l'hydrogène et $R_6$ est un groupe 3-carboxybenzoyle, caractérisé en ce qu'il consiste à faire réagir un composé de formule V tel que défini dans la revendication 3, avec une solution aqueuse de sulfure de sodium à une température inférieure à 5 ° C.

**6.** Procédé pour l'obtention des dérivés selon la revendication 1, dans lesquels $R_5$ représente un atome d'hydrogène et $R_6$ est un groupe benzoyle, éventuellement substitué par un groupe hydroxy ou un groupe nitro, caractérisé en ce qu'il consiste à faire réagir un chlorure d'acide de formule VI :

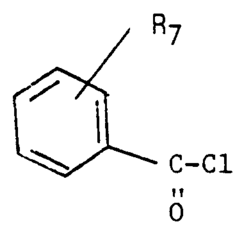

VI

dans laquelle $R_7$ est l'hydrogène, un groupe hydroxy ou un groupe nitro avec une amine de formule VII :

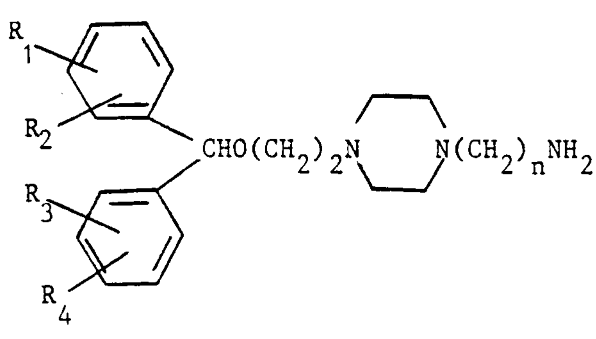

VII

dans laquelle $R_1$, $R_2$, $R_3$ et $R_4$ sont tels que définis précédemment, en présence de pyridine, dans un solvant inerte, tel qu'un hydrocarbure aromatique, par exemple le benzène ou le toluène, ou qu'un solvant chloré, par exemple le chloroforme ou le chlorure de méthylène.

**7.** Procédé selon l'une quelconque des revendications 2 à 6, caractérisé en ce que les dérivés obtenus sont transformés en leurs sels d'addition d'acides.

**8.** Utilisation des dérivés de la benzhydryloxyéthyl-pipérazine répondant à la formule générale II :

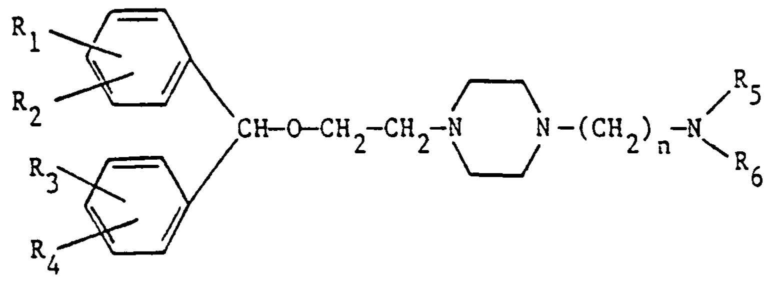

dans laquelle :

$R_1$, $R_2$, $R_3$ et $R_4$, indépendamment les uns des autres représentent un atome d'hydrogène, un atome d'halogène, un groupe alkyle inférieur, contenant 1 à 6 atomes de carbone, un groupe alcoxy inférieur contenant 1 à 6 atomes de carbone ou le groupe trifluorométhyle ;

n est un nombre entier compris entre 1 et 6 inclus ;

$R_5$ et $R_6$ représentent, l'un un atome d'hydrogène et l'autre un groupe benzoyle substitué par un groupe hydroxy, carboxy ou nitro, ou non substitué ; ou

$R_5$ et $R_6$ forment, avec l'atome d'azote auquel ils sont liés, un groupe hétérocyclique à 5 ou 6 chaînons, substitué ou non, choisi parmi les groupes ci-après : succinimidyle, 4-phénylsuccinimidyle, phtalimidyle, naphtalimidyle, phtalimidinyle, 3-hydroxyphtalimidinyle, 2-oxobenzimidazolinyle, 3-benzyl-2-oxobenzimidazolinyle, 1,2,3,4-tétrahydro-2,4-dioxoquinazolinyle, 3,4-dihydro-4-oxoquinazolinyle, 3,4-dihydro-4-oxo-2-méthylquinazolinyle, 3,7-dihydro-1,3-diméthyl-2,6-dioxo-1H-purinyle, 3,7-dihydro-3,7-diméthyl-2,6-dioxo-1H-purinyle pour la préparation des médicaments pour le traitement des états spasmodiques ou des allergies.

**Claims**

**Claims for the following Contracting States : BE, CH, DE, FR, GB, IT, LI, LU, NL, SE**

**1.** Benzhydryloxyethylpiperazine derivatives corresponding to the general formula II:

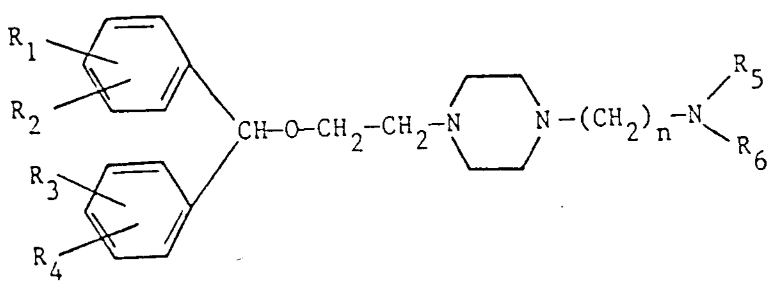

in which:

$R_1$, $R_2$, $R_3$ and $R_4$ independently of one another represent a hydrogen atom, a halogen atom, a lower alkyl group, containing 1 to 6 carbon atoms, a lower alkoxy group containing 1 to 6 carbon atoms, or the trifluorornethyl group;

n is an integer between 1 and 6 inclusive; and

one of $R_5$ and $R_6$ represents a hydrogen atom and the other is a benzoyl group, substituted by a hydroxy, carboxy or nitro group or unsubstituted;

or

$R_5$ and $R_6$ form, with the nitrogen atom to which they are bonded, a substituted or unsubstituted 5-membered or 6-membered heterocyclic group selected from the following groups: succinimidyl, 4-phenylsuccinimidyl, phthalimidyl, naphthalimidyl, phthalimidinyl, 3-hydroxyphthalimidinyl, 2-oxobenzimidazolinyl, 3-benzyl-2-oxo-benzimidazolinyl, 1,2,3,4-tetrahydro-2,4-dioxoquinazolinyl, 3,4-dihydro-4-oxoquinazolinyl, 3,4-dihydro-4-oxo-2-methylquinazolinyl, 3,7-dihdyro-1,3-dimethyl-2,6-di-oxo-1H-purinyl 3,7-dihydro-3,7-dimethyl-2,6-dioxo-1H-purinyl.

2. Process for the preparation of the derivatives according to claim 1 in which $NR_5R_6$ represents one of the following groups: succinimidyl, 4-phenylsuccinimidyl, phthalimidyl, naphthalimidyl, 2-oxobenzimidazolinyl, 3-benzyl-2-oxobenzimidazolinyl, 3,4-dihydro-4-oxoquinazolinyl, 3,4-dihydro-2-methyl-4-oxoquinazolinyl, 3,7-dihydro-1,3-dimethyl-2,6-dioxo-1H-purinyl, 3,7-dihydro-3,7-dimethyl-2,6-dioxo-1H-purinyl, or 1,2,3,4-tetrahydro-2,4-dioxoquinazolinyl, characterized in that it consists in reacting under reflux a derivative of the formula III:

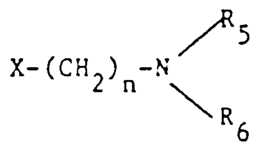

III

in which n, $R_5$ and $R_6$ are as defined in claim 1 and X is a halogeno or tosyl group, with an excess of a benzhydryloxyethylpiperazine of the formula IV:

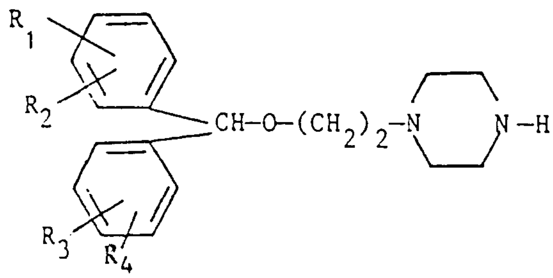

IV

in which $R_1$, $R_2$, $R_3$ and $R_4$ are as defined in claim 1, in an appropriate solvent such as toluene, xylene or methyl ethyl ketone.

3. Process for the preparation of the derivatives according to claim 1, in which $NR_5R_6$ represents the phthalimidinyl group, characterized in that it consists in reducing, in the presence of zinc and under reflux, a compound of the formula V:

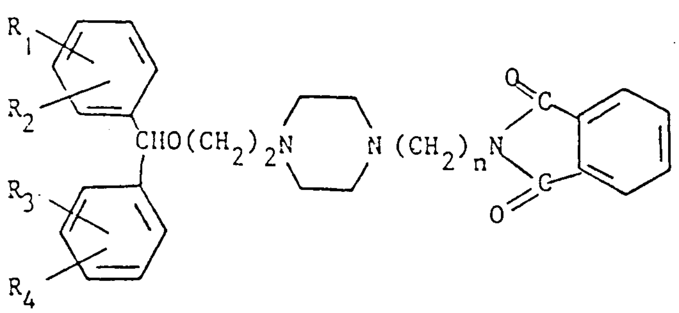

V

obtained by the process according to claim 2.

4. Process for the preparation of the derivatives according to claim 1 in which $NR_5R_6$ is the 3-hydroxyphthalimidinyl group, characterized in that it consists in reacting a compound of the formula V as defined in claim 3 with sodium borohydride in appropriate solvents, for example methanol, ethanol or isopropanol.

5. Process for the preparation of the derivatives according to claim 1 in which $R_5$ is hydrogen and $R_6$ is a

3-carboxybenzoyl group, characterized in that it consists in reacting a compound of the formula V as defined in claim 3 with an aqeuous solution of sodium sulfide at a temperature below 5 ° C.

6. Process for the preparation of the derivatives according to claim 1 in which $R_5$ represents a hydrogen atom and $R_6$ is a benzoyl group optionally substituted by a hydroxyl group or a nitro group, characterized in that it consists in reacting an acid chloride of the formula VI:

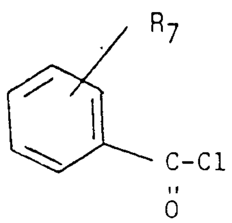

VI

in which $R_7$ is hydrogen, a hydroxy or a nitro group, with an amine of the formula VII:

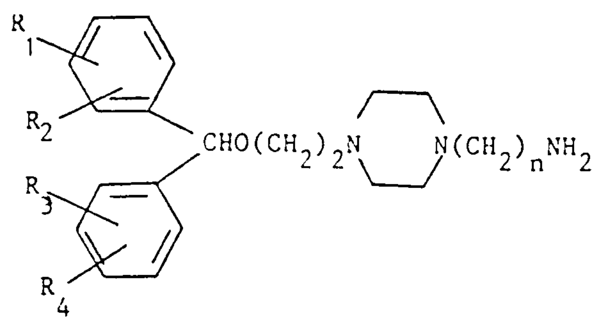

VII

in which $R_1$, $R_2$, $R_3$ and $R_4$ are as defined above, in the presence of pyridine, in an inert solvent such as an aromatic hydrocarbon, for example benzene or toluene, or such as a chlorinated solvent, for example chloroform or methylene chloride.

7. Process according to any one of claims 2 to 6, characterized in that the derivatives obtained are converted to their acid addition salts.

8. Pharmaceutical compositions, characterized in that they contain a derivative according to claim 1 as the active ingredient, in combination with a pharmaceutically acceptable vehicle.

**Claims for the following Contracting States : AT, ES**

1. Process for the preparation of the benzhydryloxyethylpiperazine derivatives corresponding to the general formula II:

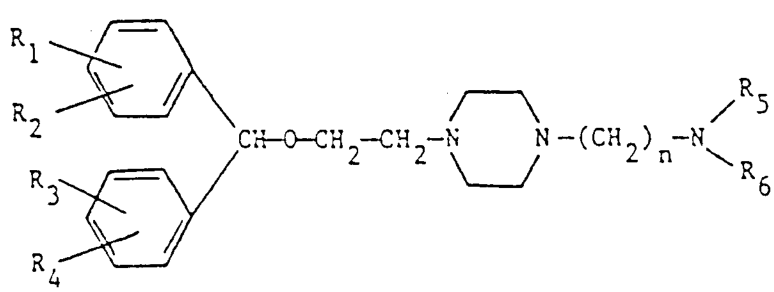

in which:
$R_1$, $R_2$, $R_3$ and $R_4$ independently of one another represent a hydrogen atom, a halogen atom, a lower alkyl group containing 1 to 6 carbon atoms, a lower alkoxy group containing 1 to 6 carbon atoms or the trifluoromethyl group;
n is an integer between 1 and 6 inclusive;
one of $R_5$ and $R_6$ represents a hydrogen atom and the other is a benzoyl group, substituted by a hydroxy, carboxy or nitro group, or unsubstituted; or
$R_5$ and $R_6$ form, with the nitrogen atom to which they are bonded, a substituted or unsubstituted 5-membered or 6-membered heterocyclic group selected from the following groups: succinimidyl, 4-phenylsuccinimidyl, phthalimidyl, naphthalimidyl, 2-oxobenzimidazolinyl, 3-benzyl-2-oxobenzimidazolinyl, 1,2,3,4-tetrahydro-2,4-dioxoquinazolinyl,3,4-dihydro-4-oxoquinazolinyl, 3,4-di-hydro-4-oxo-2-methylquinazolinyl, 3,7-dihydro-1,3-di-methyl-2,6-dioxo-1H-purinyl, 3,7-dihydro-3,7-dimethyl-2,6-dioxo-1H-purinyl, characterized in that it consists in reacting under reflux a derivative of the formula III:

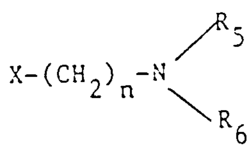

III

in which n, $R_5$ and $R_6$ are as defined above and X is a halogeno or tosyl group, with an excess of a benzhydryloxyethylpiperazine of the formula IV:

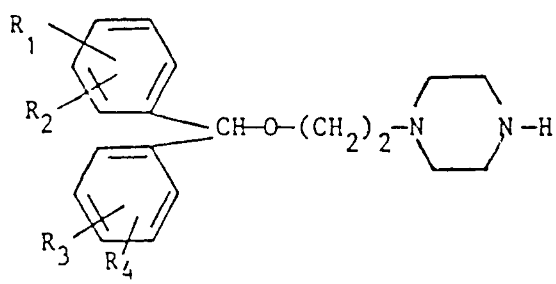

IV

in which $R_1$, $R_2$, $R_3$, and $R_4$ are as defined above, in an appropriate solvent such as toluene, xylene or methyl ethyl ketone,
and in optionally converting the derivatives obtained to their acid addition salts.

**2.** Process for the preparation of benzhydryloxyethylpiperazine derivatives corresponding to the general formula II:

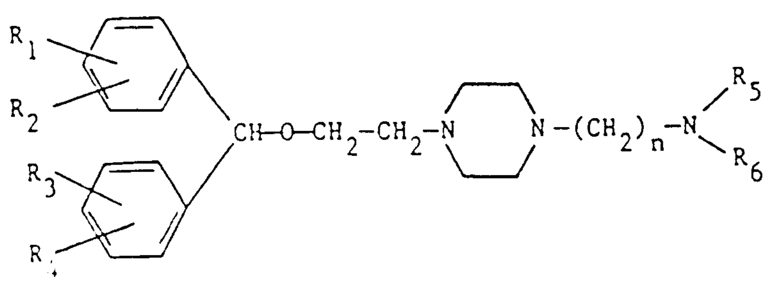

in which:
$R_1$, $R_2$, $R_3$ and $R_4$, independently of one another represent a hydrogen atom, a halogen atom, a lower alkyl group containing 1 to 6 carbon atoms, a lower alkoxy group containing 1 to 6 carbon atoms or the trifluoromethyl group;

n is an integer between 1 and 6 inclusive;
$R_5$ and $R_6$ form, with the nitrogen to which they are bonded, the phthalimidinyl group, characterized in that it consists in reducing, in the presence of zinc and under reflux, a compound of the formula V:

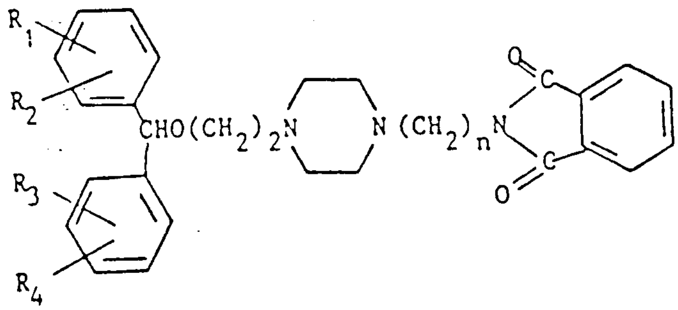

V

obtained by the process according to claim 1,
and in optionally converting the derivatives obtained into their acid addition salts.

**3.** Process for the preparation of the benzhydryloxyethylpiperazine derivatives corresponding to the general formula II:

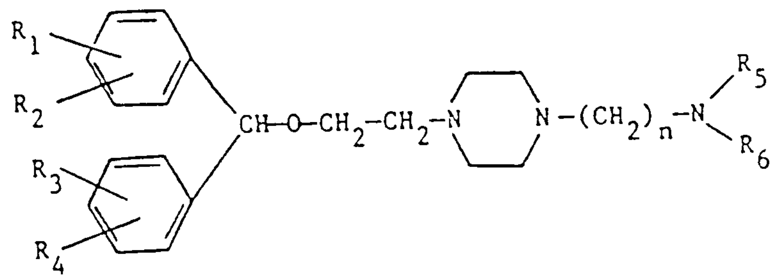

in which:
$R_1$, $R_2$, $R_3$ and $R_4$ independently of one another represent a hydrogen atom, a halogen atom, a lower alkyl group containing 1 to 6 carbon atoms, a lower alkoxy group containing 1 to 6 carbon atoms or the trifluoromethyl group;
n is an integer between 1 and 6 inclusive;
$R_5$ and $R_6$ form, with the nitrogen to which they are bonded, the 3-hydroxyphthalimidinyl group, characterized in that it consists in reacting a compound of the formula V as defined in claim 1, with sodium borohydride in appropriate solvents, for example methanol, ethanol or isopropanol,
and in optionally converting the derivatives obtained into their acid addition salts.

**4.** Process for the preparation of the benzhydryloxyethylpiperazine derivatives corresponding to the general formula II:

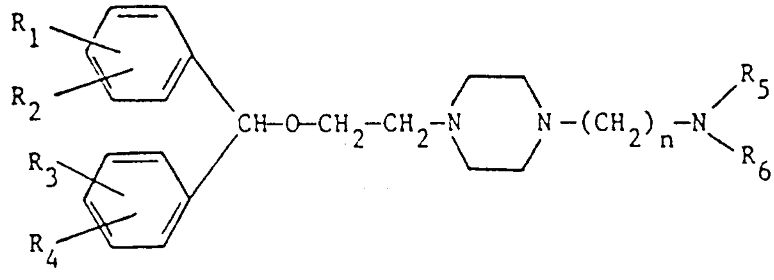

in which:
$R_1$, $R_2$, $R_3$ and $R_4$ independently of one another represent a hydrogen atom, a halogen atom, a lower alkyl group containing 1 to 6 carbon atoms, a lower alkoxy group containing 1 to 6 carbon atoms or the trifluoromethyl group;
n is an integer between 1 and 6 inclusive;
$R_5$ is hydrogen and $R_6$ is a 3-carboxybenzoyl group, characterized in that it consists in reacting a compound of the formula V as defined in claim 1, with an aqueous solution of sodium sulfide at a temperature below 5 ° C,
and in optionally converting the derivatives obtained into their acid addition salts.

**5.** Process for the preparation of the benzhydryloxyethylpiperazine derivatives corresponding to the general formula II:

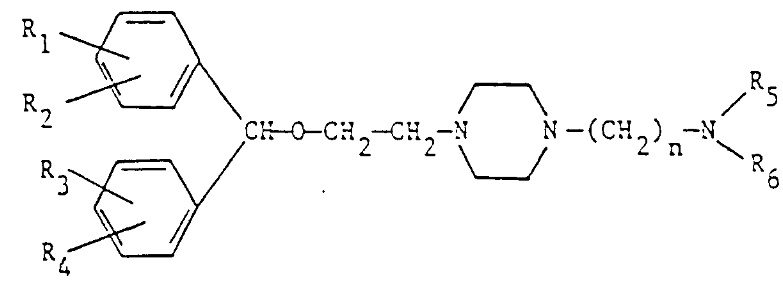

in which:
$R_1$, $R_2$, $R_3$ and $R_4$ independently of one another represent a hydrogen atom, a halogen atom, a lower alkyl group containing 1 to 6 carbon atoms, a lower alkoxy group containing 1 to 6 carbon atoms or the trifluoromethyl group;
n is an integer between 1 and 6 inclusive;
$R_5$ represents a hydrogen atom and $R_6$ is a benzoyl group optionally substituted by a hydroxyl group or a nitro group, characterized in that it consists in reacting an acid chloride of the formula VI:

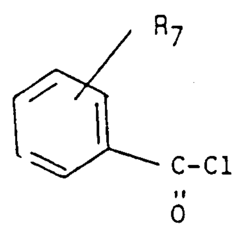

VI

in which $R_7$ is a hydrogen, a hydroxy or a nitro group, with an amine of the formula VII:

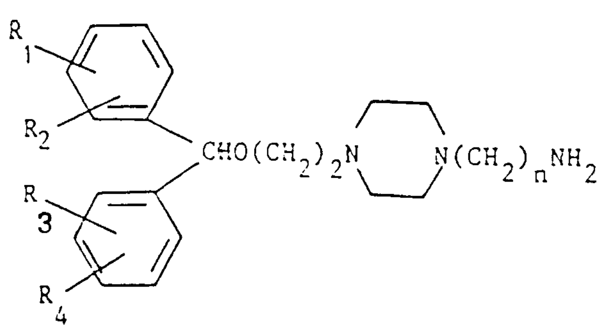

VII

in which $R_1$, $R_2$, $R_3$ and $R_4$ are as defined above, in the presence of pyridine, in an inert solvent such as an aromatic hydrocarbon, for example benzene or toluene, or such as a chlorinated solvent, for example chloroform or methylene chloride,
and in optionally converting the derivatives obtained into their acid addition salts.

**6.** Use of the benzhydryloxyethylpiperazine derivatives corresponding to the general formula II:

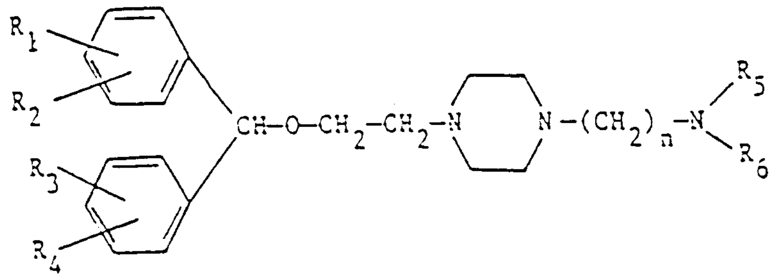

in which:
$R_1$, $R_2$, $R_3$ and $R_4$ independently of one another represent a hydrogen atom, a halogen atom, a lower alkyl group, containing 1 to 6 carbon atoms, a lower alkoxy group containing 1 to 6 carbon atoms, or the trifluoromethyl group;
n is an integer between 1 and 6 inclusive;
one of $R_5$ and $R_6$ represents a hydrogen atom and the other is a benzoyl group, substituted by a hydroxy, carboxy or nitro group or unsubstituted; or
$R_5$ and $R_6$ form, with the nitrogen atom to which they are bonded, a substituted or unsubstituted 5-membered or 6-membered heterocyclic group selected from the following groups: succinimidyl, 4-phenylsuccinimidyl, phthalimidyl naphthalimidyl, phthalimidinyl, 3-hydroxyphthalimidinyl, 2-oxobenzimidazolinyl, 3-benzyl-2-oxobenzimidazolinyl, 1,2,3,4-tetrahydro-2,4-dioxoquinazolinyl, 3,4-dihydro-4-oxoquinazolinyl, 3,4-dihydro-4-oxo-2-methylquinazolinyl, 3,7-dihdyro-1,3-dimethyl-2,6-dioxo-1H-purinyl, 3,7-dihydro-3,7-dimethyl-2,6-dioxo-1H-purinyl, for the preparation of drugs treating spasmodic states and allergies.

**Claims for the following Contracting States : GR**

**1.** Benzhydryloxyethylpiperazine derivatives corresponding to the general formula II:

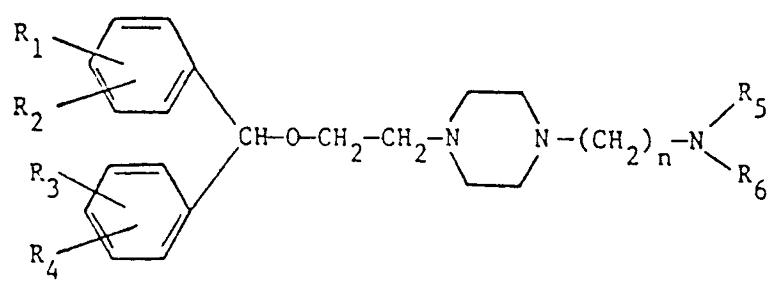

in which:
$R_1$, $R_2$, $R_3$ and $R_4$ independently of one another represent a hydrogen atom, a halogen atom, a lower alkyl group, containing 1 to 6 carbon atoms, a lower alkoxy group containing 1 to 6 carbon atoms, or the trifluoromethyl group;
n is an integer between 1 and 6 inclusive; and
one of $R_5$ and $R_6$ represents a hydrogen atom and the other is a benzoyl group, substituted by a hydroxy, carboxy or nitro group, or unsubstituted;
or

$R_5$ and $R_6$ form, with the nitrogen atom to which they are bonded, a substituted or unsubstituted 5-membered or 6-membered heterocyclic group selected from the following groups: succinimidyl, 4-phenylsuccinimidyl, phthalimidyl, naphthalimidyl, phthalimidinyl, 3-hydroxyphthalimidinyl, 2-oxobenzimidazolinyl, 3-benzyl-2-oxobenzimidazolinyl, 1,2,3,4-tetrahydro-2,4-dioxoquinazolinyl, 3,4-dihydro-4-oxoquinazolinyl, 3,4-dihydro-4-oxo-2-methylquinazolinyl, 3,7-dihdyro-1,3-dimethyl-2,6-dioxo-1H-purinyl, 3,7-dihydro-3,7-dimethyl-2,6-dioxo-1H-purinyl.

**2.** Process for the preparation of the derivatives according to claim 1 in which $NR_5R_6$ represents one of the following groups: succinimidyl, 4-phenylsuccinimidyl, phthalimidyl, naphthalimidyl, 2-oxobenzimidazolinyl, 3-benzyl-2-oxobenzimidazolinyl, 3,4-dihydro-4-oxoquinazolinyl, 3,4-dihydro-2-methyl-4-oxoquinazolinyl, 3,7-dihydro-1,3-dimethyl-2,6-dioxo-1H-purinyl, 3,7-dihydro-3,7-dimethyl-2,6-dioxo-1H-purinyl, or 1,2,3,4-tetrahydro-2,4-dioxoquinazolinyl, characterized in that it consists in reacting under reflux a derivative of the formula III:

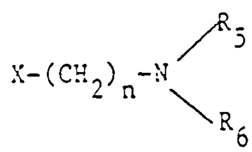

III

in which n, $R_5$ and $R_6$ are as defined in claim 1 and X is a halogeno or tosyl group, with an excess of a benzhydryloxyethylpiperazine of the formula IV:

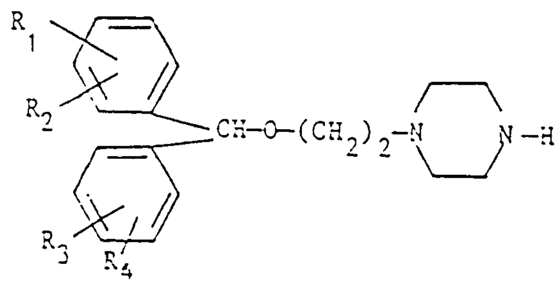

IV

in which $R_1$, $R_2$, $R_3$ and $R_4$ are as defined in claim 1, in an appropriate solvent such as toluene, xylene or methyl ethyl ketone.

**3.** Process for the preparation of the derivatives according to claim 1, in which $NR_5R_6$ represents the phthalimidinyl group, characterized in that it consists in reducing, in the presence of zinc and under reflux, a compound of the formula V:

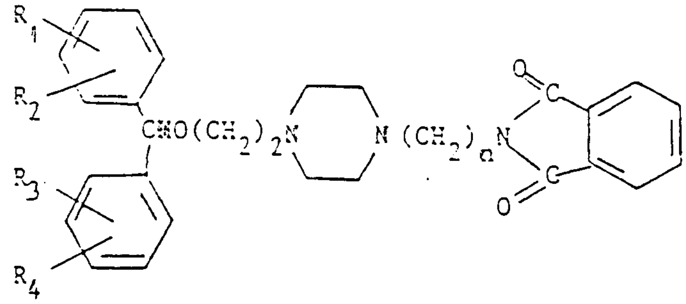

V

obtained by the process according to claim 2.

4. Process for the preparation of the derivatives according to claim 1, in which $NR_5R_6$ is the 3-hydroxyphthalimidinyl group, characterized in that it consists in reacting a compound of the formula V as defined in claim 3 with sodium borohydride in appropriate solvents, for example methanol, ethanol or isopropanol.

5. Process for the preparation of the derivatives according to claim 1, in which $R_5$ is hydrogen and $R_6$ is a 3-carboxybenzoyl group, characterized in that it consists in reacting a compound of the formula V as defined in claim 3, with an aqeuous solution of sodium sulfide at a temperature below 5 ° C.

6. Process for the preparation of the derivatives according to claim 1, in which $R_5$ represents a hydrogen atom and $R_6$ is a benzoyl group optionally substituted by a hydroxyl group or a nitro group, characterized in that it consists in reacting an acid chloride of the formula VI:

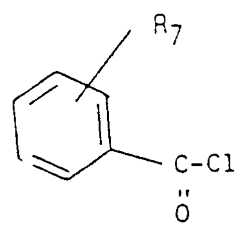

VI

in which $R_7$ is hydrogen, a hydroxy or a nitro group, with an amine of the formula VII:

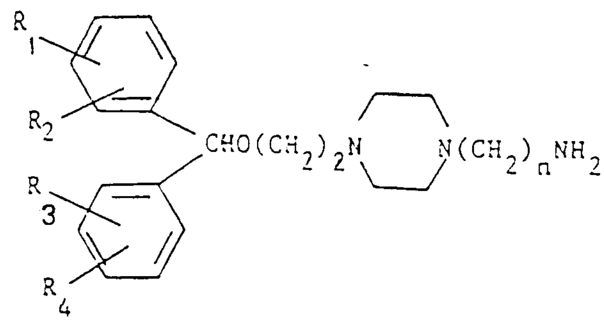

VII

in which $R_1$, $R_2$, $R_3$ and $R_4$ are as defined above, in the presence of pyridine, in an inert solvent such as an aromatic hydrocarbon, for example benzene or toluene, or such as a chlorinated solvent, for example chloroform or methylene chloride.

7. Process according to any one of claims 2 to 6, characterized in that the derivatives obtained are converted to their acid addition salts.

8. Use of the benzhydryloxyethylpiperazine derivatives corresponding to the general formula II:

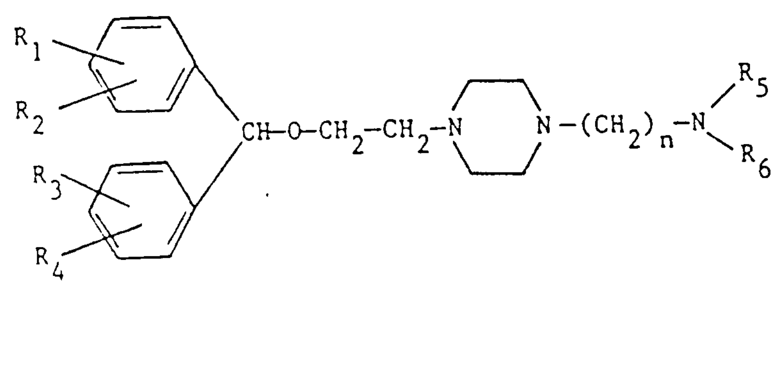

in which:
$R_1$, $R_2$, $R_3$ and $R_4$ independently of one another represent a hydrogen atom, a halogen atom, a lower alkyl group, containing 1 to 6 carbon atoms, a lower alkoxy group containing 1 to 6 carbon atoms, or the trifluoromethyl group
n is an integer between 1 and 6 inclusive
one of $R_5$ and $R_6$ represents a hydrogen atom and the other is a benzoyl group, substituted by a hydroxy, carboxy or nitro group or unsubstituted;
or
$R_5$ and $R_6$ form, with the nitrogen atom to which they are bonded, a substituted or unsubstituted 5-membered or 6-membered heterocyclic group selected from the following groups: succinimidyl, 4-phenylsuccinimidyl, phthalimidyl, naphthalimidyl, phthalimidinyl, 3-hydroxyphthalimidinyl, 2-oxobenzimidazolinyl, 3-benzyl-2-oxobenzimidazolinyl, 1,2,3,4-tetrahydro-2,4-dioxoquinazolinyl, 3,4-dihydro-4-oxoquinazolinyl, 3,4-dihydro-4-oxo-2-methylquinazolinyl, 3,7-dihdyro-1,3-dimethyl-2,6-dioxo-1H-purinyl, 3,7-dihydro-3,7-dimethyl-2,6-dioxo-1H-purinyl, for the preparation of drugs treating spasmodic states and allergies.

**Patentansprüche**
**Patentansprüche für folgende Vertragsstaaten : BE, CH, DE, FR, GB, IT, LI, LU, NL, SE**

**1.** BenzhydryloXyethyl-piperazinderivate der allgemeinen Formel II

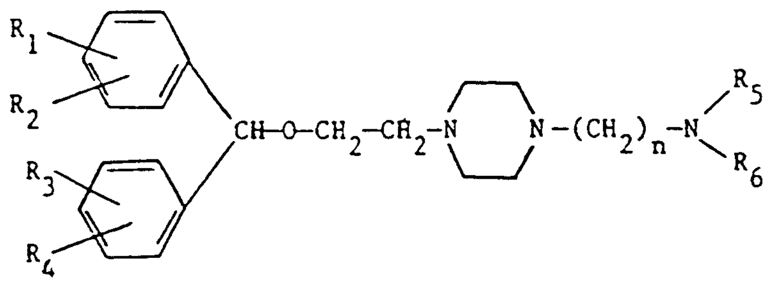

worin:
$R_1$, $R_2$, $R_3$ und $R_4$, unabhängig voneinander, ein Wasserstoffatom, ein Halogenatom, eine Niedrigalkylgruppe mit 1 bis 6 Kohlenstoffatomen, eine Niedrigalkoxygruppe mit 1 bis 6 Kohlenstoffatomen oder Trifluormethyl darstellen;
n eine ganze Zahl zwischen 1 und einschließlich 6 ist;
eines von $R_5$ und $R_6$ ein Wasserstoffatom und das andere eine gegebenenfalls durch eine Hydroxy-, Carboxy- oder Nitrogruppe substituierte Benzoylgruppe darstellt; oder
$R_5$ und $R_6$ mit dem Stickstoffatom, an das sie gebunden sind, eine gegebenenfalls substituierte 5- oder 6-gliedrige heterocyclische Gruppe bilden, ausgewählt aus den nachstehenden Gruppen: Succinimidyl, 4-Phenylsuccinimidyl, Phthalimidyl, Naphthalimidyl, Phthalimidinyl, 3-Hydroxyphthalimidinyl, 2-Oxobenzimidazolinyl, 3-Benzyl-2-oxo-benzimidazolinyl, 1,2,3,4-Tetrahydro-2,4-dioxochinazolinyl, 3,4-Dihydro-4-oxochinazolinyl, 3,4-Dihydro-4-oxo-2-methylchinazolinyl, 3,7-Dihydro-1,3-dimethyl-2,6-dioxo-1H-purinyl, 3,7-Dihydro-3,7-dimethyl-2,6-dioxo-1H-purinyl.

**2.** Verfahren zur Herstellung der Derivate nach Anspruch 1, worin $NR_5R_6$ eine der nachstehenden Gruppen darstellt: Succinimidyl, 4-Phenylsuccinimidyl, Phthalimidyl, Naphthalimidyl, 2-Oxobenzimidazolinyl, 3-Benzyl-2-oxo-benzimidazolinyl, 3,4-Dihydro-4-oxo-chinazolinyl, 3,4-Dihydro-2-methyl-4-oxochinazolinyl, 3,7-Dihydro-1,3-dimethyl-2,6-dioxo-1H-purinyl, 3,7-Dihydro-3,7-dimethyl-2,6-dioxo-1H-purinyl oder 1,2,3,4-Tetrahydro-2,4-dioxo-chinazolinyl,
dadurch gekennzeichnet, daß es darin besteht, daß ein Derivat der Formel III

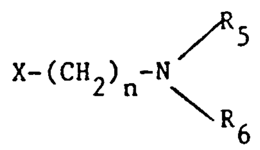

III

worin n, $R_5$ und $R_6$ wie in Anspruch 1 definiert sind und x eine Halogen- oder Tosylgruppe darstellt, unter Rückfluß mit einem Überschuß an Benzhydryloxyethyl-piperazin der Formel IV

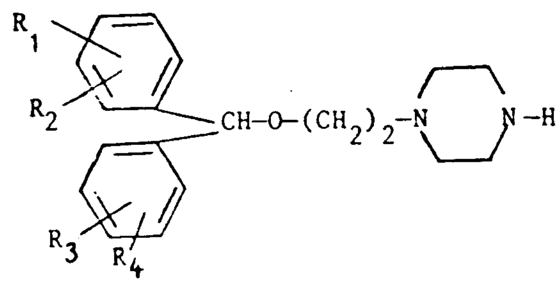

IV

worin $R_1$, $R_2$, $R_3$ und $R_4$ wie in Anspruch 1 definiert sind, in einem geeigneten Lösungsmittel, wie Toluol, Xylol oder Methylethylketon, reagieren gelassen wird.

**3.** Verfahren zur Herstellung der Derivate nach Anspruch 1, worin $NR_5R_6$ Phthalimidinyl darstellt, dadurch gekennzeichnet, daß es darin besteht, daß eine Verbindung der Formel V

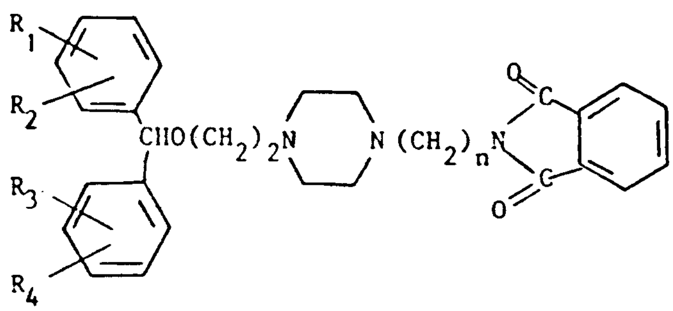

V

die durch das Verfahren gemäß Anspruch 2 erhalten ist, in Gegenwart von Zink und unter Rückfluß reduziert wird.

**4.** Verfahren zur Herstellung der Derivate nach Anspruch 1, worin $NR_5R_6$ 3-Hydroxyphthalimidinyl bedeutet, dadurch gekennzeichnet, daß es darin besteht, daß eine Verbindung der Formel V, wie in Anspruch 3 definiert, mit Natriumborhydrid in geeigneten Lösungsmitteln, wie z.B. Methanol, Ethanol oder Isopropanol, reagieren gelassen wird.

**5.** Verfahren zur Herstellung der Derivate nach Anspruch 1, worin $R_5$ Wasserstoff und $R_6$ 3-Carboxybenzoyl ist, dadurch gekennzeichnet, daß es darin besteht, daß eine Verbindung der Formel V, wie in Anspruch 3 definiert, mit einer wässerigen Natriumsulfidlösung bei einer Temperatur von weniger als 5 °C reagieren gelassen wird.

**6.** Verfahren zur Herstellung der Derivate nach Anspruch 1, worin $R_5$ ein Wasserstoffatom und $R_6$ eine gegebenenfalls durch eine Hydroxy- oder eine Nitrogruppe substituierte Benzoylgruppe ist, dadurch gekennzeichnet, daß es darin besteht, daß ein Säurechlorid der Formel VI

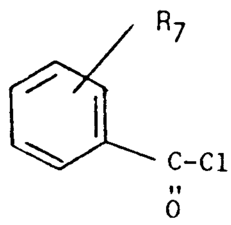

VI

worin $R_7$ Wasserstoff, eine Hydroxy- oder eine Nitrogruppe ist, mit einem Amin der Formel VII

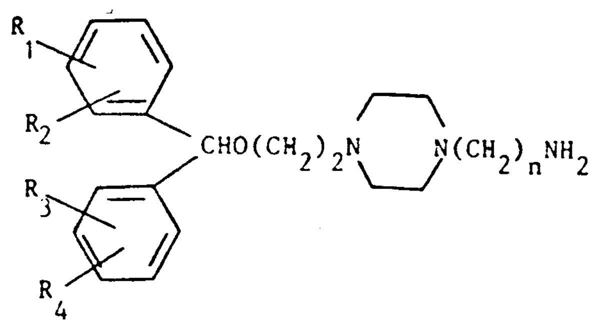

VII

worin $R_1$, $R_2$, $R_3$ und $R_4$ wie zuvor definiert sind, in Gegenwart von Pyridin in einem inerten Lösungsmittel, wie einem aromatischen Kohlenwasserstoff, z.B. Benzol oder Toluol, oder einem chlorierten Lösungsmittel, z.B. Chloroform oder Methylenchlorid, reagieren gelassen wird.

7. Verfahren nach einem der Ansprüche 2 bis 6, dadurch gekennzeichnet, daß die erhaltenen Derivate in ihre Säureadditionssalze übergeführt werden.

8. Pharmazeutische Zusammensetzungen, dadurch gekennzeichnet, daß sie als aktives Ingrediens ein Derivat nach Anspruch 1 in Kombination mit einem pharmazeutisch akzeptablen Vehikel enthalten.

**Patentansprüche für folgende Vertragsstaaten : AT, ES**

1. Verfahren zur Herstellung von Benzhydryloxyethylpiperazinderivaten der allgemeinen Formel II

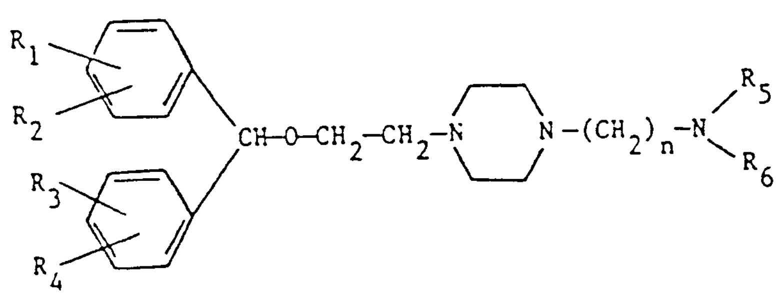

worin:
$R_1$, $R_2$, $R_3$ und $R_4$, unabhängig voneinander, ein Wasserstoffatom, ein Halogenatom, eine Niedrigalkylgruppe mit 1 bis 6 Kohlenstoffatomen, eine Niedrigalkoxygruppe mit 1 bis 6 Kohlenstoffatomen oder Trifluormethyl darstellen;
n eine ganze Zahl zwischen 1 und einschließlich 6 ist;
eines von $R_5$ und $R_6$ ein Wasserstoffatom und das andere eine gegebenenfalls durch eine Hydroxy-, Carboxy- oder Nitrogruppe substituierte Benzoylgruppe darstellt; oder
$R_5$ und $R_6$ mit dem Stickstoffatom, an das sie gebunden sind, eine gegebenenfalls substituierte 5- oder 6-gliedrige heterocyclische Gruppe bilden, ausgewählt aus den nachstehenden Gruppen: Succinimidyl,

4-Phenylsuccinimidyl, Phthalimidyl, Naphthalimidyl, 2-Oxobenzimidazolinyl, 3-Benzyl-2-oxobenzimidazolinyl, 1,2,3,4-Tetrahydro-2,4-dioxochinazolinyl, 3,4-Dihydro-4-oxochinazolinyl, 3,4-Dihydro-4-oxo-2-methylchinazolinyl, 3,7-Dihydro-1,3-dimethyl-2,6-dioxo-1H-purinyl, 3,7-Dihydro-3,7-dimethyl-2,6-dioxo-1H-purinyl, dadurch gekennzeichnet, daß es darin besteht, daß ein Derivat der Formel III

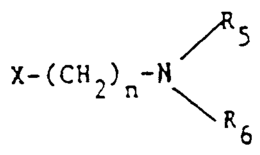

III

worin n, $R_5$ und $R_6$ wie oben definiert sind und X eine Halogen- oder Tosylgruppe darstellt, unter Rückfluß mit einem Überschuß an Benzhydryloxyethyl-piperazin der Formel IV

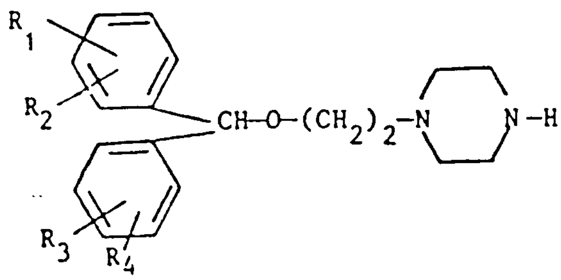

IV

worin $R_1$, $R_2$, $R_3$ und $R_4$ wie oben definiert sind, in einem geeigneten Lösungsmittel, wie Toluol, Xylol oder Methylethylketon, reagieren gelassen wird und gegebenenfalls die erhaltenen Derivate in ihre Säureadditionssalze übergeführt werden.

**2.** Verfahren zur Herstellung von Benzhydryloxyethylpiperazinderivaten der allgemeinen Formel II

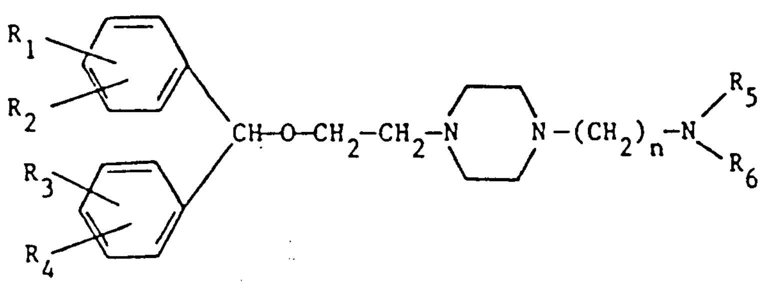

worin:
$R_1$, $R_2$, $R_3$ und $R_4$, unabhängig voneinander, ein Wasserstoffatom, ein Halogenatom, eine Niedrigalkylgruppe mit 1 bis 6 Kohlenstoffatomen, eine Niedrigalkoxygruppe mit 1 bis 6 Kohlenstoffatomen oder Trifluormethyl darstellen;
n eine ganze Zahl zwischen 1 und einschließlich 6 ist;
$R_5$ und $R_6$ mit dem Stickstoffatom, an das sie gebunden sind, Phthalimidinyl bilden, dadurch gekennzeichnet, daß es darin besteht, daß eine Verbindung der Formel V

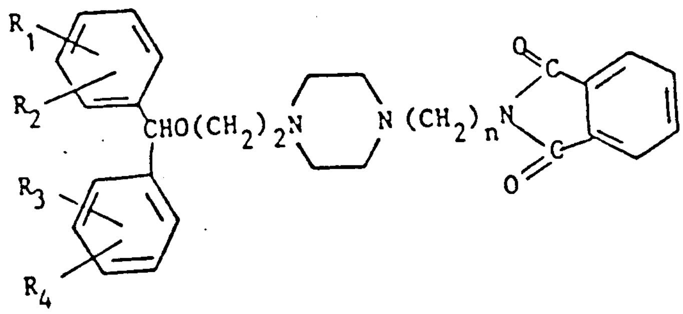

V

die durch das Verfahren gemäß Anspruch 1 erhalten ist, in Gegenwart von Zink und unter Rückfluß reduziert wird und gegebenenfalls die erhaltenen Derivate in ihre Säureadditionssalze übergeführt werden.

**3.** Verfahren zur Herstellung von Benzhydryloxyethylpiperazinderivaten der allgemeinen Formel II

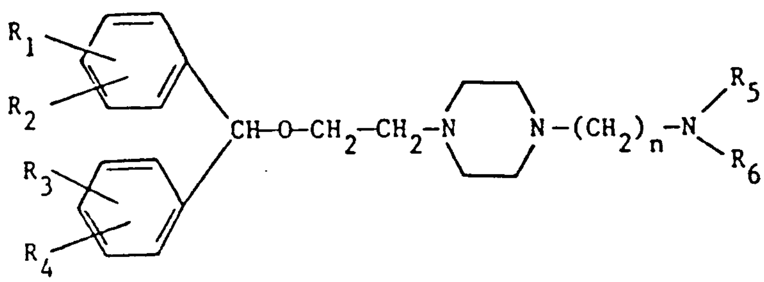

worin:
$R_1$, $R_2$, $R_3$ und $R_4$, unabhängig voneinander, ein Wasserstoffatom, ein Halogenatom, eine Niedrigalkylgruppe mit 1 bis 6 Kohlenstoffatomen, eine Niedrigalkoxygruppe mit 1 bis 6 Kohlenstoffatomen oder Trifluormethyl darstellen;
n eine ganze Zahl zwischen 1 und einschließlich 6 ist;
$R_5$ und $R_6$ mit dem Stickstoffatom, an das sie gebunden sind, 3-Hydroxyphthalimidinyl bilden, dadurch gekennzeichnet, daß es darin besteht, daß eine Verbindung der Formel V, wie in Anspruch 1 definiert, mit Natriumborhydrid in geeigneten Lösungsmitteln, wie z.B. Methanol, Ethanol oder Isopropanol, reagieren gelassen wird und gegebenenfalls die erhaltenen Derivate in ihre Säureadditionssalze übergeführt werden.

**4.** Verfahren zur Herstellung von Benzhydryloxyethylpiperazinderivaten der allgemeinen Formel II

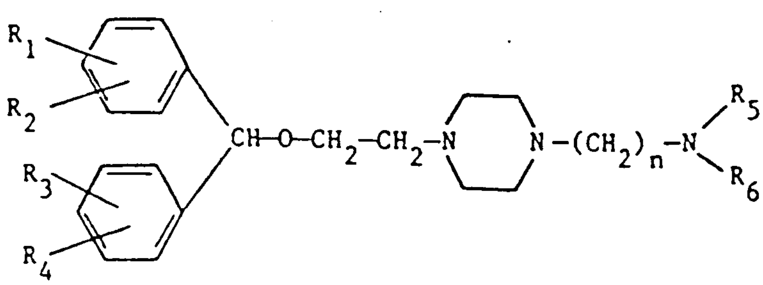

worin:
$R_1$, $R_2$, $R_3$ und $R_4$, unabhängig voneinander, ein Wasserstoffatom, ein Halogenatom, eine Niedrigalkylgruppe mit 1 bis 6 Kohlenstoffatomen, eine Niedrigalkoxygruppe mit 1 bis 6 Kohlenstoffatomen oder Trifluormethyl darstellen;
n eine ganze Zahl zwischen 1 und einschließlich 6 ist;

$R_5$ Wasserstoff ist und $R_6$ 3-Carboxybenzoyl ist, dadurch gekennzeichnet, daß es darin besteht, daß eine Verbindung der Formel V, wie in Anspruch 1 definiert, mit einer wässerigen Natriumsulfidlösung bei einer Temperatur von weniger als 5 ° C reagieren gelassen wird und gegebenenfalls die erhaltenen Derivate in ihre Säureadditionssalze übergeführt werden.

**5.** Verfahren zur Herstellung von Benzhydryloxyethylpiperazinderivaten der allgemeinen Formel II

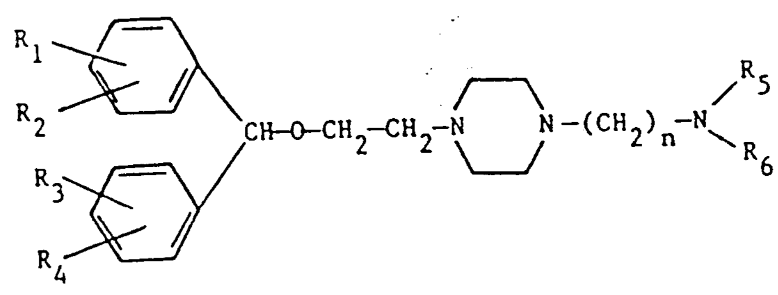

worin:
$R_1$, $R_2$, $R_3$ und $R_4$, unabhängig voneinander, ein Wasserstoffatom, ein Halogenatom, eine Niedrigalkylgruppe mit 1 bis 6 Kohlenstoffatomen, eine Niedrigalkoxygruppe mit 1 bis 6 Kohlenstoffatomen oder Trifluormethyl darstellen;
n eine ganze Zahl zwischen 1 und einschließlich 6 ist;
R5 ein Wasserstoffatom und $R_6$ eine gegebenenfalls durch eine Hydroxy- oder eine Nitrogruppe substituierte Benzoylgruppe ist, dadurch gekennzeichnet, daß es darin besteht, daß ein Säurechlorid der Formel VI

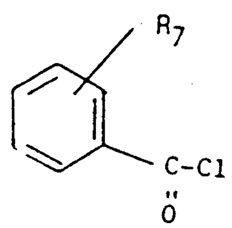

VI

worin $R_7$ Wasserstoff, eine Hydroxy- oder eine Nitrogruppe ist, mit einem Amin der Formel VII

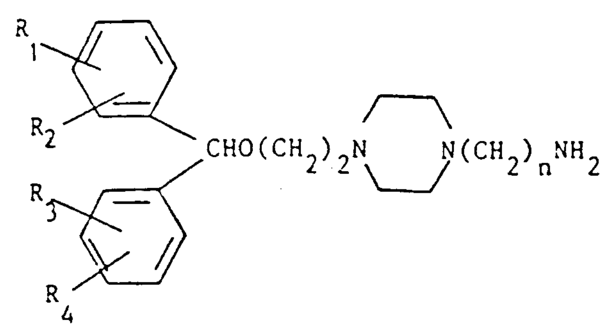

VII

worin $R_1$, $R_2$, $R_3$ und $R_4$ wie zuvor definiert sind, in Gegenwart von Pyridin in einem inerten Lösungsmittel, wie einem aromatischen Kohlenwasserstoff, z.B. Benzol oder Toluol, oder einem chlorierten Lösungsmittel, z.B. Chloroform oder Methylenchlorid, reagieren gelassen wird und gegebenenfalls die erhaltenen Derivate in ihre Säureadditionssalze übergeführt werden.

**6.** Verwendung der Benzhvdrvloxyethyl-piperazinderivate der allgemeinen Formel II:

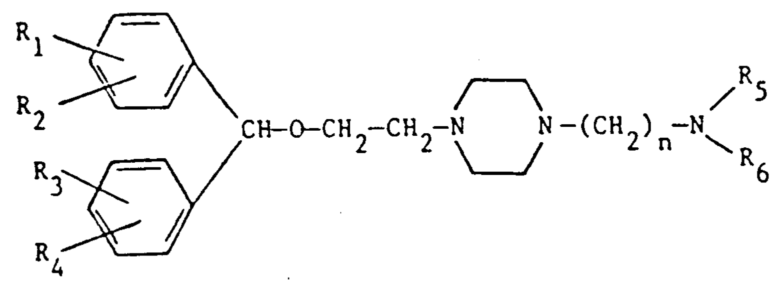

worin:
$R_1$, $R_2$, $R_3$ und $R_4$, unabhängig voneinander, ein Wasserstoffatom, ein Halogenatom, eine Niedrigalkylgruppe mit 1 bis 6 Kohlenstoffatomen, eine Niedrigalkoxygruppe mit 1 bis 6 Kohlenstoffatomen oder Trifluormethyl darstellen;
n eine ganze Zahl zwischen 1 und einschließlich 6 ist;
eines von $R_5$ und $R_6$ ein Wasserstoffatom und das andere eine gegebenenfalls durch eine Hydroxy-, Carboxy- oder Nitrogruppe substituierte Benzoylgruppe darstellt; oder
$R_5$ und $R_6$ mit dem Stickstoffatom, an das sie gebunden sind, eine gegebenenfalls substituierte 5- oder 6-gliedrige heterocyclische Gruppe bilden, ausgewählt aus den nachstehenden Gruppen: Succinimidyl, 4-Phenylsuccinimidyl, Phthalimidyl, Naphthalimidyl, Phthalimidinyl, 3-Hydroxyphthalimidinyl, 2-Oxobenzimidazolinyl, 3-Benzyl-2-oxo-benzimidazolinyl, 1,2,3,4-Tetrahydro-2,4-dioxochinazolinyl, 3,4-Dihydro-4-oxochinazolinyl, 3,4-Dihydro-4-oxo-2-methylchinazolinyl, 3,7-Dihydro-1,3-dimethyl-2,6-dioxo-1H-purinyl, 3,7-Dihydro-3,7-dimethyl-2,6-dioxo-1H-purinyl,
zur Herstellung von Medikamenten zur Behandlung von krampfartigen Zuständen oder Allergien.

**Patentansprüche für folgenden Vertragsstaat : GR**

**1.** Benzhydryloxyethyl-piperazinderivate der allgemeinen Formel II

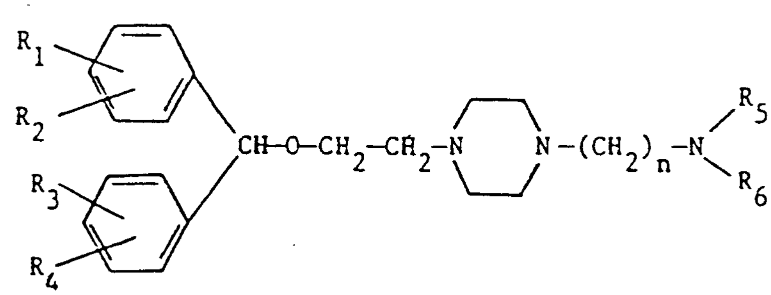

worin:
$R_1$, $R_2$, $R_3$ und $R_4$, unabhängig voneinander, ein Wasserstoffatom, ein Halogenatom, eine Niedrigalkylgruppe mit 1 bis 6 Kohlenstoffatomen, eine Niedrigalkoxygruppe mit 1 bis 6 Kohlenstoffatomen oder Trifluormethyl darstellen;
n eine ganze Zahl zwischen 1 und einschließlich 6 ist;
eines von $R_5$ und $R_6$ ein Wasserstoffatom und das andere eine gegebenenfalls durch eine Hydroxy-, Carboxy- oder Nitrogruppe substituierte Benzoylgruppe darstellt; oder
$R_5$ und $R_6$ mit dem Stickstoffatom, an das sie gebunden sind, eine gegebenenfalls substituierte 5- oder 6-gliedrige heterocyclische Gruppe bilden, ausgewählt aus den nachstehenden Gruppen: Succinimidyl, 4-Phenylsuccinimidyl, Phthalimidyl, Naphthalimidyl, Phthalimidinyl, 3-Hydroxyphthalimidinyl, 2-Oxobenzimidazolinyl, 3-Benzyl-2-oxo-benzimidazolinyl, 1,2,3,4-Tetrahydro-2,4-dioxochinazolinyl, 3,4-Dihydro-4-oxochinazolinyl, 3,4-Dihydro-4-oxo-2-methylchinazolinyl, 3,7-Dihydro-1,3-dimethyl-2,6-dioxo-1H-purinyl, 3,7-Dihydro-3,7-dimethyl-2,6-dioxo-1H-purinyl.

**2.** Verfahren zur Herstellung der Derivate nach Anspruch 1, worin $NR_5R_6$ eine der nachstehenden

Gruppen darstellt: Succinimidyl, 4-Phenylsuccinimidyl, Phthalimidyl, Naphthalimidyl, 2-Oxobenzimidazolinyl, 3-Benzyl-2-oxobenzimidazolinyl, 3,4-Dihydro-4-oxo-chinazolinyl, 3,4-Dihydro-2-methyl-4-oxochinazolinyl, 3,7-Dihydro-1,3-dimethyl-2,6-dioxo-1H-purinyl, 3,7-Dihydro-3,7-dimethyl-2,6-dioxo-1H-purinyl oder 1-2,3,4-Tetrahydro-2,4-dioxo-chinazolinyl,
dadurch gekennzeichnet, daß es darin besteht, daß ein Derivat der Formel III

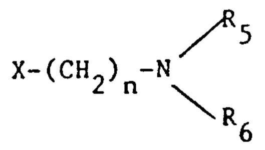

III

worin n, $R_5$ und $R_6$ wie in Anspruch 1 definiert sind und X eine Halogen- oder Tosylgruppe darstellt, unter Rückfluß mit einem Überschuß an Benzhydryloxyethyl-piperazin der Formel IV

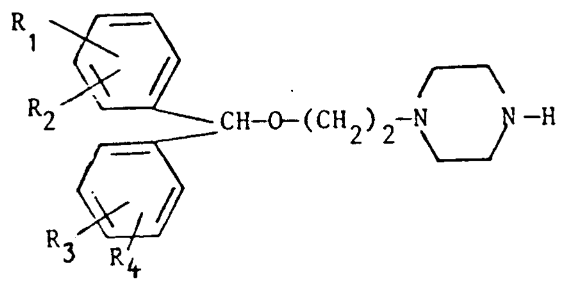

IV

worin $R_1$, $R_2$, $R_3$ und $R_4$ wie in Anspruch 1 definiert sind, in einem geeigneten Lösungsmittel, wie Toluol, Xylol oder Methylethylketon, reagieren gelassen wird.

3. Verfahren zur Herstellung der Derivate nach Anspruch 1, worin $NR_5R_6$ Phthalimidinyl darstellt, dadurch gekennzeichnet, daß es darin besteht, daß eine Verbindung der Formel V

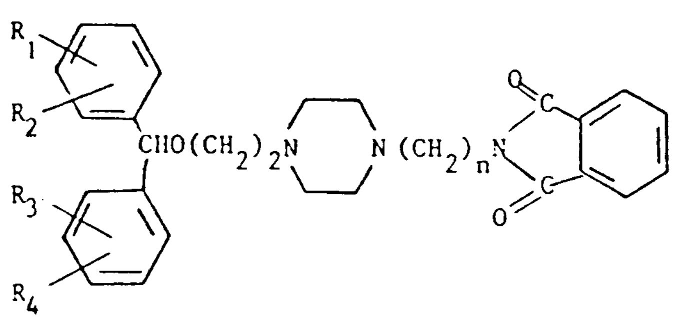

V

die durch das Verfahren gemäß Anspruch 2 erhalten ist, in Gegenwart von Zink und unter Rückfluß reduziert wird.

4. Verfahren zur Herstellung der Derivate nach Anspruch 1, worin $NR_5R_6$ 3-Hydroxyphthalimidinyl bedeutet, dadurch gekennzeichnet, daß es darin besteht, daß eine Verbindung der Formel V, wie in Anspruch 3 definiert, mit Natriumborhydrid in geeigneten Lösungsmitteln, wie z.B. Methanol, Ethanol oder Isopropanol, reagieren gelassen wird.

5. Verfahren zur Herstellung der Derivate nach Anspruch 1, worin $R_5$ Wasserstoff und $R_6$ 3-Carboxybenzoyl ist, dadurch gekennzeichnet, daß es darin besteht, daß eine Verbindung der Formel V, wie in Anspruch 3 definiert, mit einer wässerigen Natriumsulfidlösung bei einer Temperatur von weniger als 5 ° C reagieren gelassen wird.

6. Verfahren zur Herstellung der Derivate nach Anspruch 1, worin $R_5$ ein Wasserstoffatom und $R_6$ eine gegebenenfalls durch eine Hydroxy- oder eine Nitrogruppe substituierte Benzoylgruppe ist, dadurch gekennzeichnet, daß es darin besteht, daß ein Säurechlorid der Formel VI

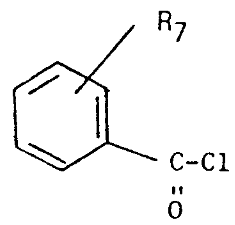

VI

worin $R_7$ Wasserstoff, eine Hydroxy- oder eine Nitrogruppe ist, mit einem Amin der Formel VII

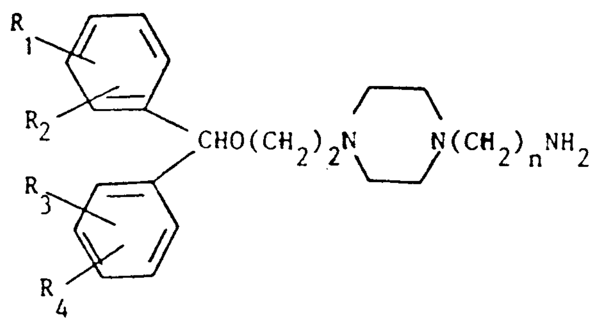

VII

worin $R_1$, $R_2$, $R_3$ und $R_4$ wie zuvor definiert sind, in Gegenwart von Pyridin in einem inerten Lösungsmittel, wie einem aromatischen Kohlenwasserstoff, z.B. Benzol oder Toluol, oder einem chlorierten Lösungsmittel, z.B. Chloroform oder Methylenchlorid, reagieren gelassen wird.

7. Verfahren nach einem der Ansprüche 2 bis 6, dadurch gekennzeichnet, daß die erhaltenen Derivate in ihre Säureadditionssalze übergeführt werden.

8. Verwendung der Benzhydryloxyethyl-piperazinderivate der allgemeinen Formel II:

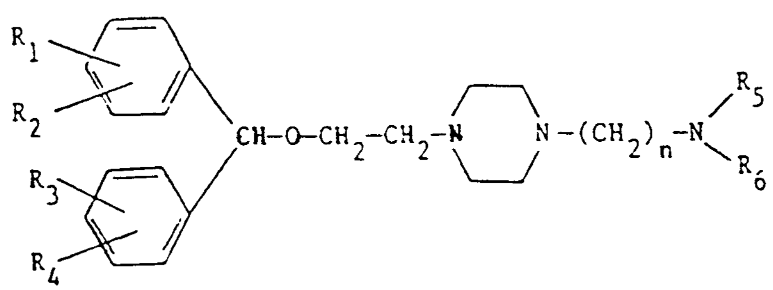

worin:
$R_1$, $R_2$, $R_3$ und $R_4$, unabhängig voneinander, ein Wasserstoffatom, ein Halogenatom, eine Niedrigalkylgruppe mit 1 bis 6 Kohlenstoffatomen, eine Niedrigalkoxygruppe mit 1 bis 6 Kohlenstoffatomen oder Trifluormethyl darstellen;
n eine ganze Zahl zwischen 1 und einschließlich 6 ist;
eines von $R_5$ und $R_6$ ein Wasserstoffatom und das andere eine gegebenenfalls durch eine Hydroxy-, Carboxy- oder Nitrogruppe substituierte Benzoylgruppe darstellt; oder
$R_5$ und $R_6$ mit dem Stickstoffatom, an das sie gebunden sind, eine gegebenenfalls substituierte 5- oder 6-gliedrige heterocyclische Gruppe bilden, ausgewählt aus den nachstehenden Gruppen: Succinimidyl,

4-Phenylsuccinimidyl, Phthalimidyl, Naphthalimidyl, Phthalimidinyl, 3-Hydroxyphthalimidinyl, 2-Oxobenzimidazolinyl, 3-Benzyl-2-oxo-benzimidazolinyl, 1,2,3,4-Tetrahydro-2,4-dioxochinazolinyl, 3,4-Dihydro-4-oxochinazolinyl, 3,4-Dihydro-4-oxo-2-methylchinazolinyl, 3,7-Dihydro-1,3-dimethyl-2,6-dioxo-1H-purinyl, 3,7-Dihydro-3,7-dimethyl-2,6-dioxo-1H-purinyl,
zur Herstellung von Medikamenten zur Behandlung von krampfartigen Zuständen oder Allergien.